(19)

**Europäisches Patentamt**
**European Patent Office**
**Office européen des brevets**

(11)  **EP 4 755 906 A2**

(12) **EUROPEAN PATENT APPLICATION**
published in accordance with Art. 153(4) EPC

(43) Date of publication:
**10.06.2026  Bulletin 2026/24**

(21) Application number: **24787537.0**

(22) Date of filing: **02.08.2024**

(51) International Patent Classification (IPC):
*C07K 14/53* (2006.01)    *C07K 14/54* (2006.01)
*A61K 38/00* (2006.01)    *A61P 29/00* (2006.01)
*C07K 19/00* (2006.01)

(52) Cooperative Patent Classification (CPC):
**C07K 14/53; A61P 29/00; C07K 14/5428;**
A61K 38/00; C07K 2319/00

(86) International application number:
**PCT/ES2024/070493**

(87) International publication number:
**WO 2025/027226 (06.02.2025 Gazette 2025/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **03.08.2023  EP 23189465**

(71) Applicants:
• **Orikine Bio SL**
  **08039 Barcelona (ES)**
• **Institució Catalana De Recerca I
  Estudis Avançats (ICREA)**
  **08010 Barcelona (ES)**
• **Fundació Centre de Regulació Genòmica**
  **08003 Barcelona (ES)**

(72) Inventors:
• **MONTERO BLAY, Ariadna Teresa**
  **08020 Barcelona (ES)**

• **SERRANO PUBUL, Luís**
  **08950 Esplugues De Llobregat (ES)**
• **CHORNY, Alejo**
  **08173 Barcelona (ES)**
• **DELGADO BLANCO, Francisco Javier**
  **08003 Barcelona (ES)**
• **RIERA BORRULL, Marta**
  **08003 Barcelona (ES)**
• **DE LA NUEZ VEULENS, Ania**
  **08003 Barcelona (ES)**

(74) Representative: **Lavoix**
  **2, place d'Estienne d'Orves**
  **75441 Paris Cedex 09 (FR)**

Remarks:
The complete document including Reference
Table(s) and the Sequence Listing(s) can be
downloaded from the EPO website

(54)  **DUAL CSF1-IL-10 CYTOKINE**

(57)  The invention relates to single chain polypeptides having IL-10 and CSF1 activities and to their use in therapy.

**EP 4 755 906 A2**

**Description**

**Field of the Invention**

The invention relates to single chain polypeptides having IL-10 and CSF1 activities and to their use in therapy.

**Background of the Invention**

Cytokines are proteins that are produced in the immune system and act as chemical messengers to communicate between cells of the immune system, and also with other cells of the body. Cytokines control the entire range of immune responses, including their initiation, type, potency, and duration. So much so that its unregulated production triggers a wide variety of inflammatory and autoimmune diseases, allergies, fibrosis, and can even lead to cancer. For this reason, there is great medical interest in the development and use of cytokines as drugs to treat diseases.

Interleukin-10 (IL-10) is an anti-inflammatory cytokine that plays a crucial role in regulating immune responses and maintaining immune homeostasis. IL-10 is a homodimeric cytokines that is produced as a monomer by various immune cells, including T cells, B cells, macrophages, and dendritic cells, and it exerts its effects by binding to its receptor complex, which is composed of two subunits, IL-10R1 and IL-10R2. When IL-10 binds to its receptor, IL-10R1 first recognizes and binds to IL-10 with high affinity, forming a complex that is then stabilized by the binding of IL-10R2. The binding of IL-10 to its receptor complex leads to the activation of signaling pathways.

When binds to its receptor on myeloid cells, IL-10 exerts potent anti-inflammatory activities, including inhibition of the production of pro-inflammatory mediators, expression of MHCII and costimulatory molecules. Simultaneously, activation of myeloid cells by IL-10 leads to the expression of anti-inflammatory mediators and promotes the development of tolerogenic phenotype, like M2-like anti-inflammatory macrophages and tolerogenic dendritic cells. In contrast to the strong anti-inflammatory activities on myeloid cells, IL-10 is a growth factor for B cells and promotes plasma cell differentiation and antibody production. Similarly, IL-10 has stimulatory effects on CD8+ T cells, inducing their proliferation, INFY production, and cytotoxicity.

In humans, there is a strong genetic association between the IL-10 pathway and autoimmune diseases, such as inflammatory bowel disease. Loss-of-function mutations in IL-10, IL-10RA, or IL-10RB result in early-onset, therapy-resistant severe enterocolitis. Restoring IL-10RA or IL-10 expression by hematopoietic stem cell transplantation rapidly alleviates clinical symptoms. In turn, mice deficient in IL-10 or IL-10R develop colitis spontaneously, while the administration of IL-10 in different animal models of colitis has been shown to be consistently beneficial. Finally, mice in which all cells of the organism respond to IL-10, except specific subsets of myeloid cells that includes monocytes and macrophages, also develop spontaneous colitis that is mediated by the production of the clinically validated target IL-23.

Myeloid cells exclusively express CSF1 receptor, i.e. CSF1R. Stimulation of CSF1R by its ligand, the cytokine CSF1, is essential for the differentiation, proliferation and maintenance of M2-like anti-inflammatory macrophages in the intestine, which play a key role in generating a tolerogenic environment through the production of IL-10. In autoimmune disease, the M2-like anti-inflammatory macrophages of the affected tissues, such as the mucosa of IBD patients, are replaced by inflammatory monocytes that drive disease progression.

While in preclinical studies IL-10 has shown promising results in ameliorating inflammation and tissue damage in models of colitis, clinical trials investigating the therapeutic potential of IL-10 in patients with colitis have not been successful. One possible explanation is that the delivery of IL-10 to the site of inflammation may be a limiting factor. However, some strategies aimed to enhance the delivery of IL-10 at the site of inflammation did not show clinical benefit (AMT-101).

Another possible explanation is because the anti-inflammatory effects of IL-10 on myeloid cells are counteracted by pro-inflammatory tissue-damaging effects, including activation, differentiation, and induction of antibodies by B cells and induction of cytotoxic activities by CD8+ T cells.

To address this limitation, there is a need for IL-10-based therapeutics that can selectively activate myeloid cells while avoiding activation of other immune cell subsets. Several strategies have been proposed for developing IL-10-based therapeutics that selectively activate myeloid cells. These include fusion of IL-10 to antibodies that bind to receptors on myeloid cells, or encapsulation of IL-10 in nanocarriers (liposomes or nanoparticles) that are specifically targeted to myeloid cells

The present invention seeks to overcome or at least alleviate one or more of the deficiencies in the prior art.

**Summary of the Invention**

The invention relates to a single chain polypeptide having IL-10 and CSF1 activities.

The invention further relates to a pharmaceutical composition comprising the single chain polypeptide according to the invention.

The invention further relates to the single chain polypeptide according to the invention for use as a medicament, in particular for use for treating an inflammatory disease.

## Detailed description of the invention

The inventors have designed single chain polypeptides combining IL-10 and CSF1 activities. Fusion proteins combining IL-10 and CSF1 monomers display reduced IL-10 activity when linking the N-terminus of IL-10 to the C-terminus of CSF1, or dramatically reduced IL-10 activity when linking the C-terminus of IL-10 to the N-terminus of CSF1. Unexpectedly, the design of fusion proteins combining single chain dimeric IL-10 and CSF1 monomer made it possible to obtain single chain polypeptides displaying substantial IL-10 and CSF1 activities.

On one hand, IL-10 monomers pair to form a 'swapped-domain' dimeric IL-10 protein. In a swapped-domain dimeric protein like IL-10, the monomeric structure of the standard IL-10 helix bundle cytokine opens and embraces in an antiparallel fashion another IL-10 opened monomer generating two functional split cytokine domains together, defining two adjacent 3D domains. On the other hand, CSF1 monomers dimerise, with the two monomeric CSF1 molecules face to face and hold together by a disulphide bond.

Without wishing to be bound by a theory, the inventors hypothesized that fusion of IL-10 and CSF1 could result in the formation of concatemers since IL-10 is a swapped dimer and CSF1 dimerizes, which would be prevented when IL-10 monomer is replaced by a single chain dimeric IL-10.

The functional characterization of the dual single chain polypeptide having IL-10 and CSF1 activities has shown that the dual CSF1-IL-10 cytokine inhibits activation of myeloid cells and induces differentiation of monocyte-derived macrophages that have a regulatory phenotype (M2 phenotype). Furthermore, the dual CSF1-IL-10 cytokine has specificity for monocytes and does not activate CD8 T cells or B cells.

Functionally, in vivo, the dual CSF1-IL-10 cytokine was shown to alleviate the clinical symptoms of inflammatory disease, in a TNBS-induced colitis mouse model.

## Single chain polypeptide having IL-10 and CSF1 activities

A single chain polypeptide having IL-10 and CSF1 activities is thus provided.

As used herein 'IL-10 activity" or "IL-10 activities" denotes one or more biological activities mediated by IL-10 through binding to its receptor, IL-10R. These comprise or consist of (i) the binding to IL-10R on myeloid cells, in particular monocytes, or (ii) anti-inflammatory activities, or preferentially both. Anti-inflammatory activities include (a) the inhibition of production of pro-inflammatory mediators (e.g. pro-inflammatory cytokines such as tumour necrosis factor-$\alpha$ (TNF-$\alpha$), IL-1, IL-12, IL-6 and granulocyte-macrophage colony-stimulating factor, inflammatory enzymes such as cyclo-oxygenase 2 and inducible nitric oxide synthase, chemokines such as RANTES, membrane inflammatory protein-1$\alpha$ (MIP1$\alpha$), IL-8, and eotaxin), and/or (b) the inhibition of expression of MHCII and costimulatory molecules by myeloid cells, and/or (c) the induction of differentiation of monocytes into tolerogenic macrophages. In some embodiments, anti-inflammatory activities comprise inhibition of production of TNF-$\alpha$ and/or IL-6 by activated monocytes. In some embodiments, anti-inflammatory activities comprise induction of differentiation of monocytes into tolerogenic macrophages. Tolerogenic macrophages have M2-like phenotype and can be identified by their regulatory phenotype and high levels of expression of CD206 and CD163. In some embodiments, IL-10 activities comprise or consist of binding to IL-10R on myeloid cells, in particular monocytes, inhibition of production of TNF-$\alpha$ and/or IL-6 by activated monocytes, and differentiation of monocytes into tolerogenic macrophages.

In some embodiments, the single chain polypeptide having IL-10 and CSF1 activities has reduced T cells and B cell activation capacities compared to IL-10.

As used herein "CSF1 activity" or "CSF1 activities" denotes one or more biological activities mediated by activation of CSF1 receptor, CFS1-R. These comprise or consist of (i) the activation of CFS1-R at the surface of monocytes or progenitors thereof, and/or (ii) the differentiation of monocytes into macrophages, preferentially both. In some embodiments, activation of CSF1-R is triggered by binding of CSF1 to CSF1-R.

In some embodiments, the single chain polypeptide having IL-10 and CSF1 activities has similar or higher affinity to CSF1R compared to wild-type CSF1. In some embodiments, the single chain polypeptide having IL-10 and CSF1 activities has reduced affinity to IL-10 receptor $\alpha$ (IL10RA) and IL-10 receptor $\beta$ (IL10RB) compared to wild-type IL-10. In some embodiments, the single chain polypeptide having IL-10 and CSF1 activities has similar or higher affinity to CSF1R compared to wild-type CSF1 and has reduced affinity to IL-10 receptor $\alpha$ (IL10RA) and IL-10 receptor $\beta$ (IL10RB) compared to wild-type IL-10. These properties are expected to favor selective binding of the single chain polypeptide having IL-10 and CSF1 activities to myeloid cells that exclusively express CSF1 receptor, among cells of the innate immune system, and subsequent activation thereof.

By 'similar affinity' it is meant herein the affinity preferably does not vary (i.e. increase or decrease) by more than a 5-fold, preferably 4-fold, 3-fold, 2-fold, or 1.5-fold factor compared to the reference level of affinity of the wild-type cytokine (CSF1).

By 'higher' or 'reduced' it is meant herein the affinity preferably varies (i.e. increase or decrease) by at least a 5-fold, preferably, 7-fold, 10-fold, 15-fold, 20-fold, 30-fold, 40-fold, 50-fold, 100-fold, 200-fold factor compared to the reference level of affinity of the wild-type cytokine (CSF1 or IL-10 as appropriate).

In a first aspect, the single chain polypeptide having IL-10 and CSF1 activities is thus provided that comprises an IL-10 monomer fused to a CSF1 monomer wherein the N-terminus of IL-10 is linked to the C-terminus of CSF1 trough a linker.

Indeed, in this orientation, the single chain polypeptide has decreased IL-10 activity compared to wild-type IL-10, but maintain a CSF1 activity similar to wild-type CSF1.

According to this aspect, the single chain polypeptide having IL-10 and CSF1 activities is thus comprises, in the N-terminus to C-terminus direction, a CSF1 monomer, a peptide linker, and an IL-10 monomer.

Preferably, the linker bridging the C-terminus of CSF1 monomer to the N-terminus of IL-10 monomer is a (flexible) peptide sequence composed of Gly and Ser residues, in different proportions. Examples of suitable linkers comprise or consist of GGGSGGSGGSGGSGGSGGSGGSGGG (25 amino acid long, SEQ ID NO: 34), GGGGGSGGSGGSGGSGGSGGSGGSGGSGGG (30 amino acid long, SEQ ID NO: 35), or GGGGGSGGGGSGGSGGSGGSGGSGGSGGSGGSGGG (35 amino acid long, SEQ ID NO: 36).

The CSF1 monomer may be as defined in the below section.

The IL-10 monomer may be wild-type mature IL-10 as shown in SEQ ID NO:11, or a mutant thereof, or a polypeptide comprising the sequence of mature IL-10 and on the N-terminal end 1, 2, 3 or more additional amino acids residues consecutively present in the signal peptide of II-10 (i.e. A, RA or VRA).

In some embodiments, the single chain polypeptide having IL-10 and CSF1 activities comprises or consists of sequence SEQ ID NO: 24, SEQ ID NO: 25, SEQ ID NO: 26, or a sequence at least 80%, at least 85%, at least 90%, at least 95%, at least 98%, at least 99% or 100% identical thereto and that retains IL-10 and CSF1 activities.

In a second aspect, a single chain polypeptide having IL-10 and CSF1 activities is provided that comprises a single chain dimeric IL-10 fused to a CSF1 monomer.

In some embodiments, the single chain polypeptide having IL-10 and CSF1 activities comprises a single chain (SC) dimeric IL-10 fusion protein. In some embodiments, the single chain polypeptide having IL-10 and CSF1 activities comprises a CSF1 monomer-single chain dimeric IL-10 fusion protein.

In some embodiments the single chain dimeric IL-10 and CSF1 monomer are linked through a linker that comprises from 10 to 45 amino acid residues, preferably 12 to 40 amino acids, still preferably 15 to 40 amino acids, still preferably 20 to 35 amino acids, even more preferably 25 to 35 amino acids.

Preferably, the linker bridging the C-terminus of SC dimeric IL-10 to the N-terminus of CSF1, or bridging the N-terminus of SC dimeric IL-10 to the C-terminus of CSF1, is a (flexible) peptide sequence composed of Gly and Ser residues, in different proportions. Examples of suitable linkers comprise or consist of GGSGGSGGSGGSGGG (15 amino acid long, SEQ ID NO: 33), GGGSGGSGGSGGSGGSGGSGGSGGG (25 amino acid long, SEQ ID NO: 34), GGGGGSGGSGGSGGSGGSGGSGGSGGSGGG (30 amino acid long, SEQ ID NO: 35), or GGGGGSGGGGSGGSGGSGGSGGSGGSGGSGGSGGG (35 amino acid long, SEQ ID NO: 36).

Preferably, when the single chain polypeptide having IL-10 and CSF1 activities comprises a CSF1 monomer-single chain dimeric IL-10 fusion protein, the linker bridging the C-terminus of CSF1 to the N-terminus of SC dimeric IL-10 comprises more than 15 amino acids, preferably at least 20 or 25 amino acids.

*CSF1 monomer*

In said first and second aspects of the invention, the CSF1 monomer incorporated into the single chain polypeptide having IL-10 and CSF1 activities may be a wild-type CSF1 monomer, or mutant thereof, or a circular permutant CSF1 monomer.

A polypeptide sequence of wild-type human CSF1 is shown in SEQ ID NO: 12.

In some embodiments, the CSF1 monomer is a mutant CSF1 or a circular permutant CSF1 monomer that has similar or higher affinity to CSF1R compared to wild-type CSF1.

In some embodiments, the CSF1 monomer is a CSF1 monomer mutant that comprises sequence SEQ ID NO:12 modified by at least substitution(s):

    a) Q17R;
    b) V78W;
    c) T124I;
    d) V120I;
    e) Q17R, T124I and V120I;
    f) V78W, T124I and V120I;or

g) Q17R, V78W, T124I and V120I.

Substitution Q17R or V78W in CSF1 monomer mutant was shown to improve EC-50 values in HEK293 cells transfected with a CSF1R/reporter kit (hence affinity for CSF1R) compared to wild-type CSF1. Substitution T124I or V120I in CSF1 monomer mutant are engineered to increase stability of the CSF1 monomer in the conformation bound to the receptor CSF1R and they increase the maximum response of HEK cells transformed with the CSFR1 reporter kit.

Circular permutation in a protein structure is a rearrangement of the amino acid sequence, such that the original N- and C-termini of the polypeptide seem to be linked and new ones created elsewhere. Hence, circular permutants are generally prepared by connecting the native protein termini, or terminal regions, via a covalent linker and introducing new N- and C-terminal ends through the cleavage of an existing peptide bond.

In some embodiments, the CSF1 monomer is a CSF1 circular permutant generated by linking an amino acid in each of the N- and C-terminal regions of CSF-1 and by suppressing the peptide bond consisting of residues at positions 95 to 99 or SEQ ID NO: 12.

As used herein, an amino acid in each of the N- and C-terminal regions of CSF-1 denotes amino acids localised at the N-terminus of CSF1 (i.e. residue at position 1 of SEQ ID NO:12) and at the C-terminus terminus of CSF1 (i.e. residue at position 150 of SEQ ID NO:12), or at an amino acid position located at most 2, 3, 4, 5, 6 ,7 or 8 residues apart from the N- or C-terminus of CSF1.

Preferably, the CSF1 monomer is a CSF1 circular permutant that comprises or consists of sequence SEQ ID NO: 9 (so-called ORKMCSF_013), or SEQ ID NO: 10 (so-called ORKMCSF_014), or a sequence at least 80%, at least 85%, at least 90%, at least 95%, at least 98%, at least 99% or 100% identical thereto and that retains CSF1 activity. Both circular permutants were shown to activate the downstream CSF1 signaling cascade with similar EC-50 values and slightly better than the wild-type CSF1 monomer.

*Single chain dimeric IL-10*

The SC dimeric IL-10 incorporated into the single chain polypeptide having IL-10 and CSF1 activities may be a fusion protein comprising a first IL-10 monomer fragment comprising at least $\alpha$-helices A to F of IL-10, a peptide linker, and a second IL-10 monomer fragment comprising at least $\alpha$-helices A to F of IL-10, or a circular permutant thereof.

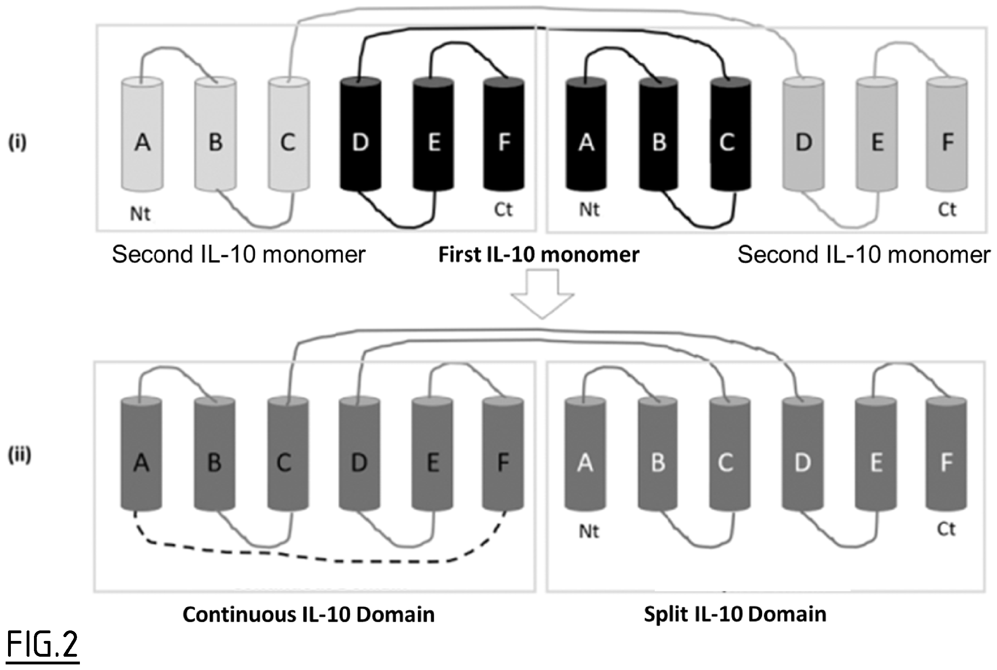

The folding of the SC dimeric IL-10 is such that a 'continuous 3D IL-10 domain' is formed (left-hand domain in Figure 2), and a split 3D IL-10 domain is formed (right-hand domain in Figure 2).

A polypeptide sequence of wild-type human IL-10 is shown in SEQ ID NO: 11:

```
SPGQGTQSENSCTHFPGNLPNMLRDLRDAFSRVKTFFQMKDQLDNLLLKESLLEDFKGYLGCQALSEMIQFYLEEVMPQ
          hhh      hhhhhhhhhh                     hhhhhh     hhhhhhhhhhhhhhhhhhhh
                HA                                                        HB


AENQDPDIKAHVNSLGENLKTLRLRLRRCHRFLPCENKSKAVEQVKNAFNKLQEKGIYKAMSEFDIFINYIEAYMTMKIRN
hhh    hhhhhhhhhhhhhhhhhhh             hhhhhhhhhhh   hhhhhhh   hhhhhhhhhhhhhhh
        HC                                HD         HE        HF
```

Positions of the helices are represented by strings of 'h' in the above sequence. For $\alpha$-helix F, depending of the structural assignments done by different software on different crystal structures of the same molecule, the helix consistently extends up to $M_{154}$, and may extends up to $M_{156}$ or $I_{158}$ (characters underlined weavy). Accordingly, it is herein considered that $\alpha$-helix F is entirely incorporated as long as the IL-10 monomer fragments comprise amino acid residues extending at least up to $M_{154}$.

According to an embodiment, SC dimeric IL-10 comprises or consists of sequence

```
MTQSENSCTHFPGNLPNMLRDLRDAFSRVKTFFQMKDQLDNLLLKESLLEDFKGYLGCQALSEMIQFYLEEVMPQ
        hhh     hhhhhhhhhhh                   hhhhhh      hhhhhhhhhhhhhhhhh
                                 HA                                           HB
AENQDPDIKAHVNSLGENLKTLRLRLRRCHRFLPCENKSKAVEQVKNAFNKLQEKGIYKAMSEFDIFINY
hhh     hhhhhhhhhhhhhhhhhh              hhhhhhhhhhh    hhhhhhh    hhhhhh
          HC                                    HD         HE       HF
IEAYX(NtCt)ZNMLRDLRDAFSRVKTFFQMKDQLDNLLLKESLLEDFKGYLGCQALSEMIQFYLEEV
hhhh          hhhhhhhhhh                 hhhhhh      hhhhhhhhhhhhhhhh
HF              HA                                                  HB
MPQAENQDPDIKAHVNSLGENLKTLRLRLRRCHRFLPCENKSKAVEQVKNAFNKLQEKGIYKAMSEFDIF
hhhhhh     hhhhhhhhhhhhhhhhhhh              hhhhhhhhhhh    hhhhhhh    hhh
             HC                                     HD         HE
INYIEAYMTMKIRN
hhhhhhhhhhhhh
    HF
```

(sequence SEQ ID NO: 65-X-(NtCt)-Z-SEQ ID NO: 66),

wherein (NtCt) is a peptide linker,

wherein X is absent or present, and where present it consists in one or more amino acids of the original IL-10 sequence in continuity with the preceding amino acids on its N-terminal side, optionally with a mutation to accommodate the NtCt linker,

wherein Z is absent or present, and where present it consists one or more amino acids of the original IL-10 sequence in continuity with the preceding amino acids on its C-terminal side, optionally with a mutation to accommodate the NtCt linker, or a sequence at least 80%, at least 85%, at least 90%, at least 95%, at least 98%, at least 99% or 100% identical thereto and that retain at least the same stability, and/or at least the same level of interaction with IL-10 receptor.

For instance X may represent M, MT, MTM, MTMN (SEQ ID NO: 67) that are present in the original IL-10 sequence in continuity with the preceding amino acids on its N-terminal side. X may also represent L or K, i.e. mutation compared to the original IL-10 sequence to accommodate the NtCt linker. Accordingly, X is preferably selected from the group consisting of M, MT, MTM, MTMN (SEQ ID NO: 67), L or K.

For instance Z may represent LP that are present in the original IL-10 sequence in continuity with the following amino acids on its C-terminal side. Z may also represent PEFA (SEQ ID NO: 68) or ELA. Accordingly, Y is preferably selected from the group consisting of LP, PEFA (SEQ ID NO: 68) or ELA.

The NtCt linker sequence bridging the C-terminus of one IL-10 monomer fragment and the N-terminus of the other IL-10 monomer fragment may have any suitable sequence. It preferentially comprises a sequence that defines a conformationally restrained structure, i.e. a 'structured' linker. In this way, the engineered linker helps to conformationally restrain the 3D domain structures in an appropriate / desired structural orientation. Such linkers may have from about 3 to about 20 amino acid residues; from about 3 to about 16 amino acid residues; from about 4 to about 12 amino acid residues, from about 4 to about 8 amino acid residues, from about 3 to 8 amino acid residues or from about 3 to 6 amino acid residues. Beneficially, such linkers do not contain a plurality of Gly and/or Ser residues adjacent each other; for example, beneficially the linker peptide contains 5 or less adjacent Gly and/or Ser residues; suitably 3 or less adjacent Gly and/or Ser residues; 2 or less Gly and/or Ser residues; contains only isolated Gly and/or Ser residues; or in some embodiments contains no Gly and/or Ser residues. In an embodiment, the NtCt linker comprises from 3 to 20 amino acid residues and comprises no more than 2 adjacent Gly and/or Ser residues.

According to some embodiments, the NtCt linker comprises sequence NGGLDY (SEQ ID NO: 30), FGGLDY (SEQ ID NO: 48), YKTIT (SEQ ID NO: 31), or DKDIRDGD (SEQ ID NO: 32).

The NtCt linker may be preceded, at its N-terminal end, by X, i.e. amino acid residue(s) that are naturally and consecutively present at position(s) 152 up to 157 of IL-10 as shown in SEQ ID NO: 11. Alternatively, or additionally the NtCt linker may be followed, at its C-terminal end, by Z, i.e. amino acid residue(s) that are naturally and consecutively present at position(s) 16 up to 20 of IL-10 as shown in SEQ ID NO: 11.

Hence, according to some embodiments, the NtCt linker and the X or Z sequence at its N-terminal side or C-terminal side (X and Z sequences in bold have the same structure as in IL10 and they correspond to the WT IL10 sequence or could have point mutations to accommodate the linker) comprises or consists of sequence **NNGGLDYL** (SEQ ID NO: 71), NFGGLDYL (SEQ ID NO: 45), **LYKTITPEFA** (SEQ ID NO: 46), **KDKDIRDGDELA** (SEQ ID NO: 47).

In some embodiments, the single chain dimeric IL-10 comprises or consists of SEQ ID NO: 2 (ORK10-002), SEQ ID NO: 3 or SEQ ID NO: 49 (ORK10-003; with or without the addition of a HiBit Tag sequence for quantification at the C-terminus of SEQ ID NO:3), or SEQ ID NO: 4 (ORK10-005), or a sequence at least 80%, at least 85%, at least 90%, at least 95%, at least 98%, at least 99% or 100% identical thereto and that retains IL-10 activity.

In some embodiments, the single chain dimeric IL-10 comprises a first IL-10 monomer fragment that includes a substitution D84E at the residue naturally present at position 84 of SEQ ID NO:11. The single chain dimeric IL-10 may then comprise or consist of SEQ ID NO: 1 or SEQ ID NO: 20 (Foldikine10 with or without the addition of a HiBit Tag sequence for quantification at the C-terminus of SEQ ID NO:1).

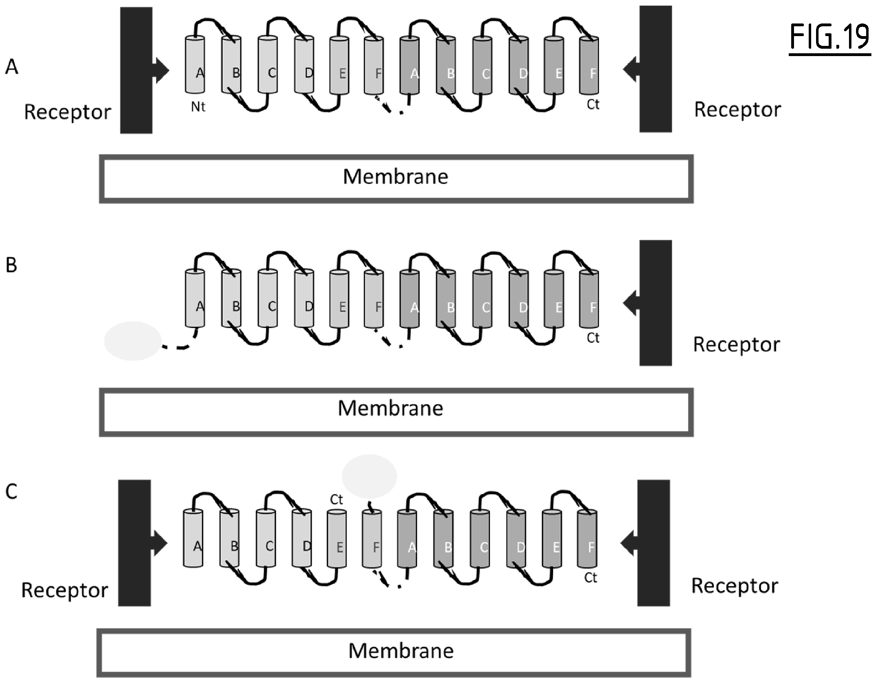

According to some embodiments, the SC dimeric IL-10 consists of a circular permutant of one of the above SC dimeric IL-10. It is thought that the use of a circular permutant could be advantageous in order to minimize a possible masking effect due to the way the fused CSF1 monomer is connected to the SC dimeric IL-10 (Figure 19).

In some embodiments, the circular permutant comprises a first IL-10 monomer fragment comprising α-helices E to F of IL-10, a first SC dimeric IL-10 peptide linker, and a second IL-10 monomer fragment comprising at least α-helices A to F of IL-10, a second peptide linker, and a third IL-10 monomer fragment comprising at least α-helices A to D of IL-10. In some embodiments, the circular permutant comprises sequence SEQ ID NO: 5 (Foldikine10_cut116, also called herein Foldikine10_cp116), or a sequence at least 80%, at least 85%, at least 90%, at least 95%, at least 98%, at least 99% or 100% identical thereto and that retains IL-10 activity.

*Resulting single chain polypeptide comprising a single chain dimeric IL-10 fused to a CSF1 monomer and having IL-10 and CSF1 activities*

According to some embodiments, the single chain polypeptide having IL-10 and CSF1 activities thus comprises or consists of sequence SEQ ID NO: 14, SEQ ID NO:16, SEQ ID NO: 17, SEQ ID NO:18, SEQ ID NO:19, SEQ ID NO:1, SEQ ID NO:20, SEQ ID NO: 21, SEQ ID NO: 22, or a sequence at least 80%, at least 85%, at least 90%, at least 95%, at least 98%, at least 99% or 100% identical thereto and that retains IL-10 and CSF1 activities.

Pharmaceutical composition

The invention further relates to a pharmaceutical composition comprising the single chain polypeptide having IL-10 and CSF1 activities and a pharmaceutically acceptable carrier.

A suitable pharmaceutically acceptable 'carrier' (such as diluents, adjuvants, excipients or vehicles) is described in "Remington's Pharmaceutical Sciences" by E. W. Martin. Pharmaceutical compositions of the invention are formulated to conform to regulatory standards and can be administered orally, intravenously, topically, or via other standard routes. As used herein, the term 'carrier' includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase 'pharmaceutically acceptable' or 'pharmacologically-acceptable' refers to molecular entities and compositions that do not produce an allergic or similar untoward reaction when administered to a human or a non-human mammal.

Acceptable pharmaceutical vehicles can be liquids, such as water and oils, including those of petroleum, animal, vegetable or synthetic origin, such as peanut oil, soybean oil, mineral oil, sesame oil and the like. The pharmaceutical vehicles can be saline, gum acacia, gelatin, starch paste, talc, keratin, colloidal silica, urea, and the like. **In** addition, auxiliary, stabilising, thickening, lubricating and colouring agents may be used. When administered to a subject, the pharmaceutically acceptable vehicles are preferably sterile. Water is a suitable vehicle particularly when the compound of the invention is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or buffering agents.

The medicaments and pharmaceutical compositions of the invention can take the form of liquids, solutions, suspensions, lotions, gels, tablets, pills, pellets, powders, modified-release formulations (such as slow or sustained-release), suppositories, emulsions, aerosols, sprays, capsules (for example, capsules containing liquids or powders), liposomes, microparticles or any other suitable formulations known in the art. Other examples of suitable pharmaceutical vehicles are described in Remington's Pharmaceutical Sciences, Alfonso R. Gennaro ed., Mack Publishing Co. Easton, Pa., 19th ed., 1995, see for example pages 1447-1676.

**In** some embodiments the therapeutic compositions or medicaments of the invention are formulated in accordance with routine procedures as a pharmaceutical composition adapted for oral administration (more suitably for human beings). Compositions for oral delivery may be in the form of tablets, lozenges, aqueous or oily suspensions, granules, powders, emulsions, capsules, syrups, or elixirs, for example. Thus, in various embodiments, the pharmaceutically acceptable vehicle may be a capsule, tablet or pill.

Orally administered compositions may contain one or more agents, for example, sweetening agents such as fructose, aspartame or saccharin; flavouring agents such as peppermint, oil of wintergreen, or cherry; colouring agents;

and preserving agents, to provide a pharmaceutically palatable preparation. When the composition is in the form of a tablet or pill, the compositions may be coated to delay disintegration and absorption in the gastrointestinal tract, so as to provide a sustained release of active agent over an extended period of time. Selectively permeable membranes surrounding an osmotically active driving compound are also suitable for orally administered compositions. In these dosage forms, fluid from the environment surrounding the capsule is imbibed by the driving compound, which swells to displace the agent or agent composition through an aperture. These dosage forms can provide an essentially zero order delivery profile as opposed to the spiked profiles of immediate release formulations. A time delay material such as glycerol monostearate or glycerol stearate may also be used. Oral compositions can include standard vehicles such as mannitol, lactose, starch, magnesium stearate, sodium saccharine, cellulose, magnesium carbonate, etc. Such vehicles are preferably of pharmaceutical grade. For oral formulations, the location of release may be the stomach, the small intestine (the duodenum, the jejunem, or the ileum), or the large intestine. The person skilled in the art is able to prepare formulations that will not dissolve in the stomach, yet will release the material in the duodenum or elsewhere in the intestine. Suitably, the release will avoid the deleterious effects of the stomach environment, either by protection of the peptide (or derivative) or by release of the peptide (or derivative) beyond the stomach environment, such as in the intestine. To ensure full gastric resistance a coating impermeable to at least pH 5.0 would be essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac, which may be used as mixed films.

To aid dissolution of the therapeutic agent(s) into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. Potential nonionic detergents that could be included in the formulation as surfactants include: lauromacrogol 400, polyoxyl 40 stearate, polyoxyethylene hydrogenated castor oil 10, 50 and 60, glycerol monostearate, polysorbate 20, 40, 60, 65 and 80, sucrose fatty acid ester, methyl cellulose and carboxymethyl cellulose. These surfactants, when used, could be present in the formulation of the peptide or nucleic acid or derivative either alone or as a mixture in different ratios.

Typically, compositions for intravenous administration comprise sterile isotonic aqueous buffer. Where necessary, the compositions may also include a solubilising agent.

Mixtures of the polypeptides as described herein may be prepared in water suitably mixed with one or more excipients, carriers, or diluents. Dispersions may also be prepared in glycerol, liquid polyethylene glycols, and mixtures thereof and in oils. Under ordinary conditions of storage and use, these preparations may contain a preservative to prevent the growth of microorganisms. The pharmaceutical forms suitable for injectable use include sterile aqueous solutions or dispersions and sterile powders for the extemporaneous preparation of sterile injectable solutions or dispersions. In all cases the form may be sterile and may be sufficiently fluid to enable injection by an appropriate syringe. Suitably, the composition is stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For parenteral administration in an aqueous solution, for example, the solution may be suitably buffered if necessary and the liquid diluent first rendered isotonic with sufficient saline or glucose. These particular aqueous solutions are especially suitable for intravenous, intramuscular, subcutaneous, intratumoral and intraperitoneal administration. In this connection, sterile aqueous media that can be employed will be known to those of skill in the art.

Another suitable route of administration for the pharmaceutical compositions of the invention is via pulmonary or nasal delivery.

Additives may be included to enhance cellular uptake of a therapeutic agent of the invention, such as the fatty acids, oleic acid, linoleic acid and linolenic acid.

Therapeutic applications

The single chain polypeptide having IL-10 and CSF1 activities of the invention, or pharmaceutical composition comprising it, is for use as a medicament.

Accordingly, the invention further relates to a method of treatment which comprises administering the single chain polypeptide having IL-10 and CSF1 activities of the invention to a subject in need thereof.

The subject may be a human or non-human mammal such as a rodent (mouse or rat), a primate (e.g. a monkey), a pig, etc.

The subject in need thereof is in particular a subject suffering from an inflammatory disease. The single chain polypeptide having IL-10 and CSF1 activities is designed to preferentially activate myeloid cells (monocytes, macrophages, dendritic cells) that co-express CSF1R and IL-10R - and that induce anti-inflammatory responses - over CD8 T cells and B cells expressing IL-10R that could induce cytotoxic responses and auto-antibodies when activated.

Accordingly, the invention further relates to a single chain polypeptide according the invention for use for treating an inflammatory disease. Also provided is a method of treating an inflammatory disease which comprises administering the single chain polypeptide having IL-10 and CSF1 activities of the invention to a subject in need thereof.

In some embodiments, the inflammatory disease is an inflammatory disease of the gut such as inflammatory bowel disease, including Crohn's disease and colitis, Behçet's disease, and/or other autoimmune diseases including but not limited to rheumatoid arthritis or diabetes mellius type 1, systemic lupus erythematosus, among others.

Treatment regimens may vary, and often depend on the type and/or location of the disease, the stage / progression of the disease, and/or the health and age of the patient. The skilled clinician will be able to determine a suitable therapeutic treatment regime under each circumstance.

The invention will be further illustrated in view of the following figures and examples.

## FIGURES

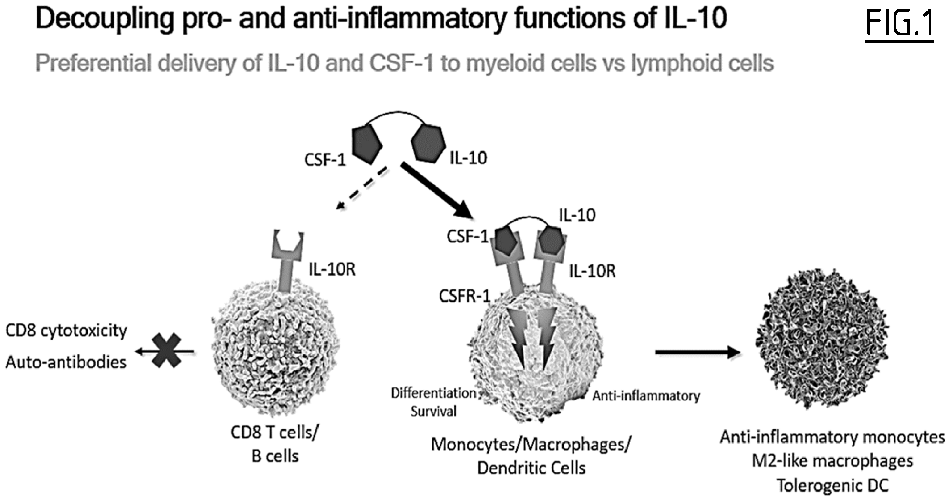

**Figure 1. Decoupling pro and anti-inflammatory functions of IL10.** The figure shows an engineered single molecule that brings together IL10 and CSF1 activities where the binding affinity for the IL-10 receptor is weakened and, as a result, only cells expressing the CSF1 receptor will be activated by IL-10.

**Figure 2. Schematic illustration for forming foldikine based on a swapped domain dimeric class II cytokine (e.g. IL-10):** (i) wild-type domain swapped dimer showing $\alpha$-helices D, E and F of each IL-10 monomer unit aligning with the opposing IL-10 monomer $\alpha$-helices A, B and C, to form adjacent 3D IL-10 domains comprising $\alpha$-helices A, B and C of a first IL-10 monomer and $\alpha$-helices D, E and F of a second IL-10 monomer; (ii) single-chain foldikine-10 based on fused wild-type IL-10 monomers in which monomer sequences are joined to make a single chain polypeptide by linking the C-terminus of one IL-10 monomer sequence to the N-terminus of a second monomer sequence using a peptide linker (dashed line). Wild-type linkers shown in solid lines. Boxes indicate the $\alpha$-helices that form each of the left and right 3D IL-10 domains (Continuous IL-10 domain and Split IL-10 Domain).

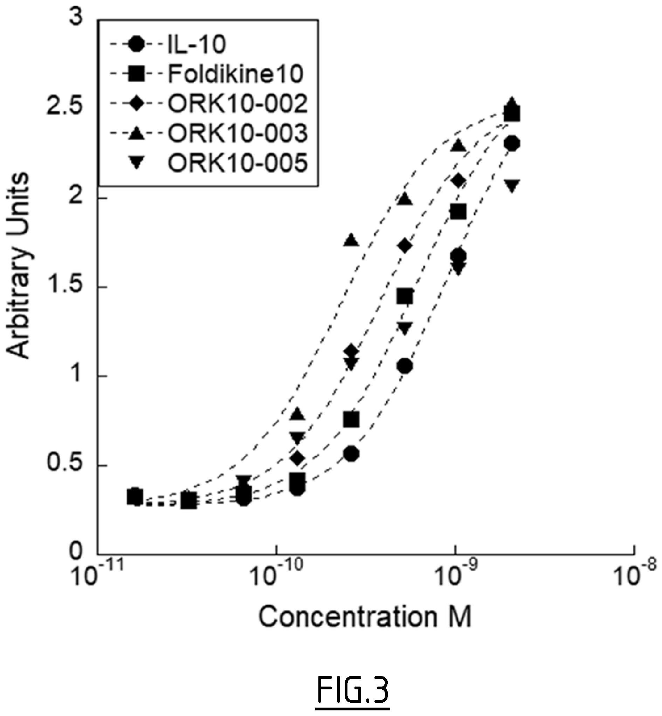

**Figure 3. Analysis of versions of Foldikine-10.** The different versions are tag-less and have been quantified by ELISA (IL-10). Activity of the designed molecules have been assessed in HEK-Blue10 commercial cells in a dose-response experiment and the data fitted to a sigmoidal dose-response to determine the EC-50 (see Methods). We have fixed the baseline to 0.27 and the Hill slope to -1.6. IL-10WT (SEQ ID NO:11), Foldikine10 (SEQ ID NO: 1), ORK10-002 (SEQ ID NO: 2), ORK10-003 (SEQ ID NO: 3) and ORK10-005 (SEQ ID NO: 5).

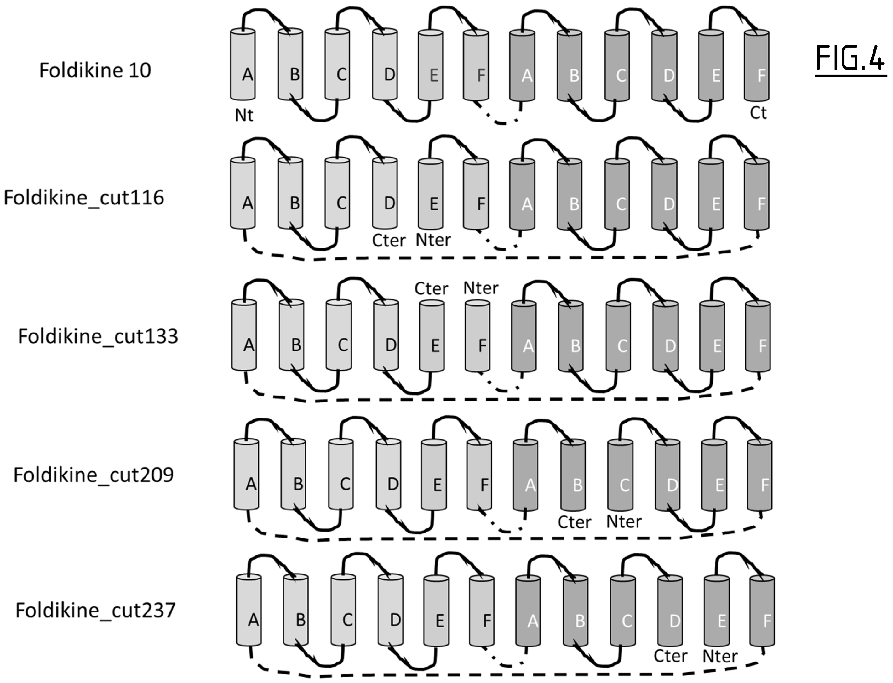

**Figure 4. Circular permutants of Foldikine-10.** Schematic illustration of the circular permutations of foldikine-10 are depicted. Wild-type linkers shown in solid lines. Boxes indicate the $\alpha$-helices that form each of the left and right 3D IL-10 domains (Continuous IL-10 domain and Split IL-10 Domain). Discontinuous lines represent the engineered NtCt linkers. The new Nt and Ct resulting from opening the loops are shown as Nter and Cter. SEQ ID NO: 5 Foldikine_cut116, SEQ ID NO: 6 Foldikine_cut133, SEQ ID NO: 7 Foldikine_cut209, SEQ ID NO: 8 Foldikine_cut237.

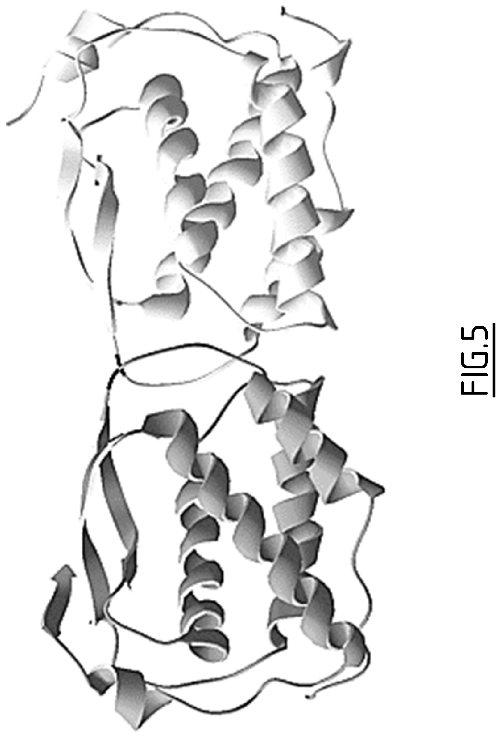

**Figure 5. CSF1 dimeric structure.** CSF1 dimeric complex with the two subunits in different shades of gray. The position of the disulfide bridge is shown in black.

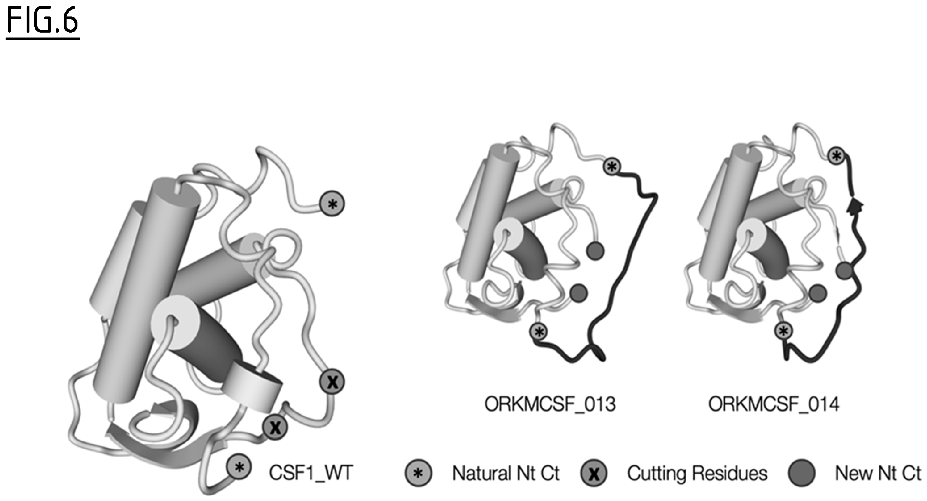

**Figure 6. Circular Permutants of CSF1.** CSF1 Natural Nt-Ct and the two residues where we opened the loop (Y95, K100). Two circular permutants (ORKMCSF_013 and ORKMCSF_014) are generated with natural Nt-Ct linked with two different linkers.

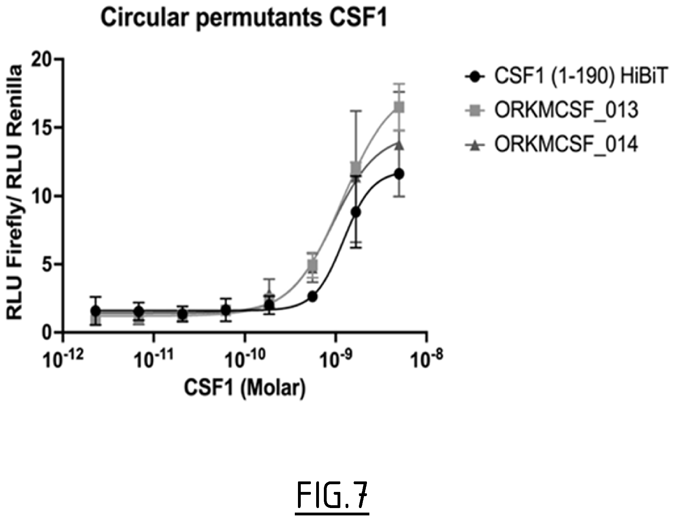

**Figure 7. Circular Permutants of CSF1** assessed in HEK293 cells transfected with a reporter cell kit for measuring the MAPK/ERK downstream signaling cascade after CSF1/CSF1R activation. CSF1 (1-190) HiBit corresponds to SEQ ID NO: 51. ORKMCSF_013 is SEQ ID NO: 9 and ORKMCSF_014 is SEQ ID NO: 10.

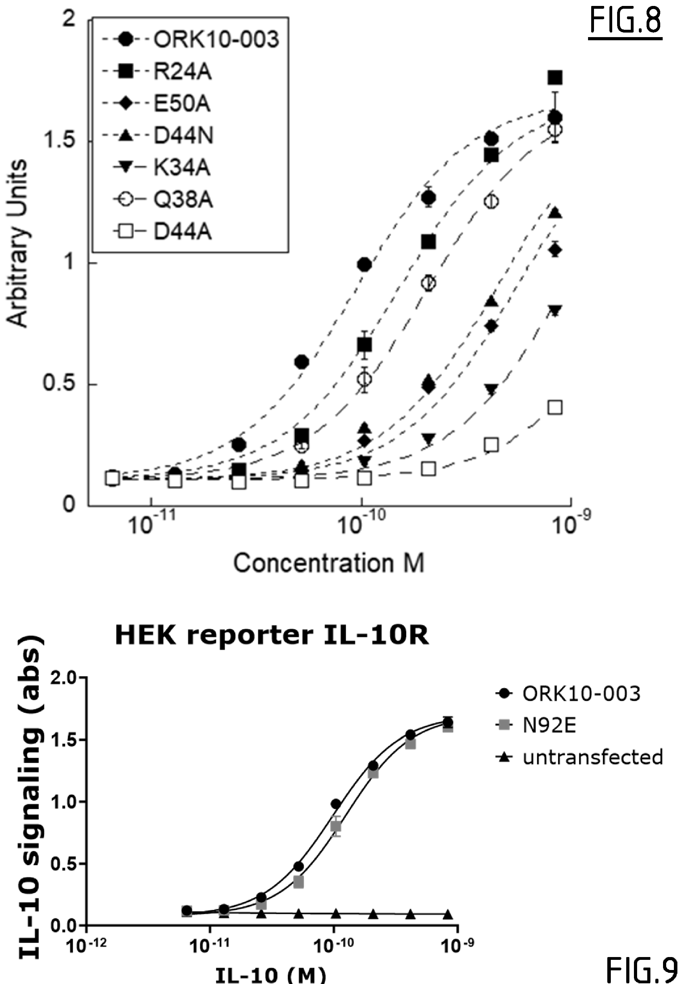

**Figure 8. Dose-response analysis of IL-10 variants with engineered affinity towards IL10RA in HEK-Blue10 cells.** All the mutations were only introduced in one halve (continuous domain) of the ORK10-003b foldikine scaffold (SEQ ID NO: 49). The data has been fitted to a sigmoidal dose-response to determine the EC-50 (see Methods). We have fixed the basal and saturation values. Foldikine10 (ORK10-003REF is SEQ ID NO: 49 ORK10-003-HiBit). The numbering of the mutants is based on SEQ ID NO: 50. R24 is R163, K34 is 173, Q38 is 177, D44 is 183, E50 is 189 in ORK10-003b. We fixed the baseline to 0.27 and the Hill slope to -1.6.

**Figure 9. Dose-response analysis of IL-10 variants with engineered affinity towards IL10RB in HEK-Blue10**

**cells.** All the mutations were only introduced in one halve (continuous domain) of the ORK10-003b foldikine scaffold (SEQ ID NO: 49). The data has been fitted to a sigmoidal dose-response to determine the EC-50 (see Methods). We have fixed the basal and saturation values. The numbering of the mutants is based on SEQ ID NO: 50. N92 is 231 in ORK10-003b. We fixed the baseline to 0.12 and -1.9 for the last two rows.

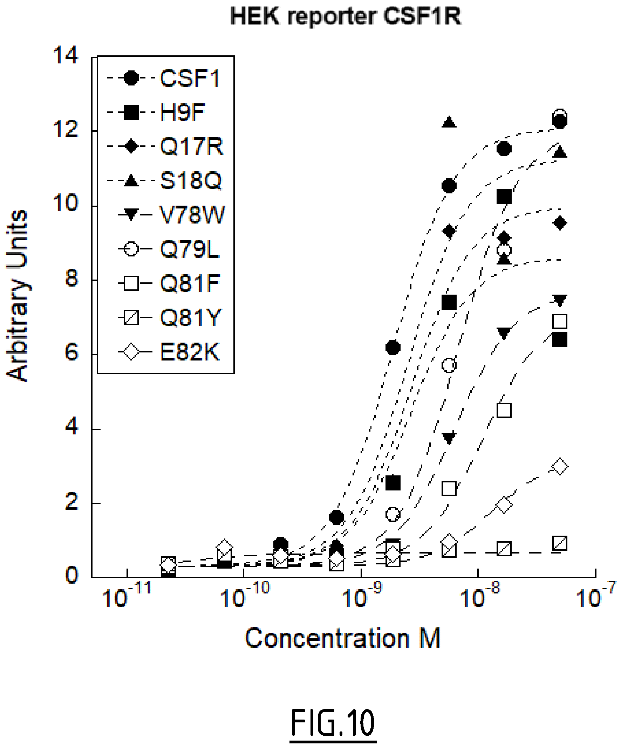

**Figure 10. Dose response analysis in HEK reporter cells for CSF1** (SEQ ID NO: 12) **predicted improved affinity mutants.** We show the titration curve and in the bottom the table with the EC-50 values. The numbering of the mutants is based on SEQ ID NO: 12.The data has been fitted to a sigmoidal dose-response to determine the EC-50 (see Methods). We have fixed the basal values and the Hill slope.

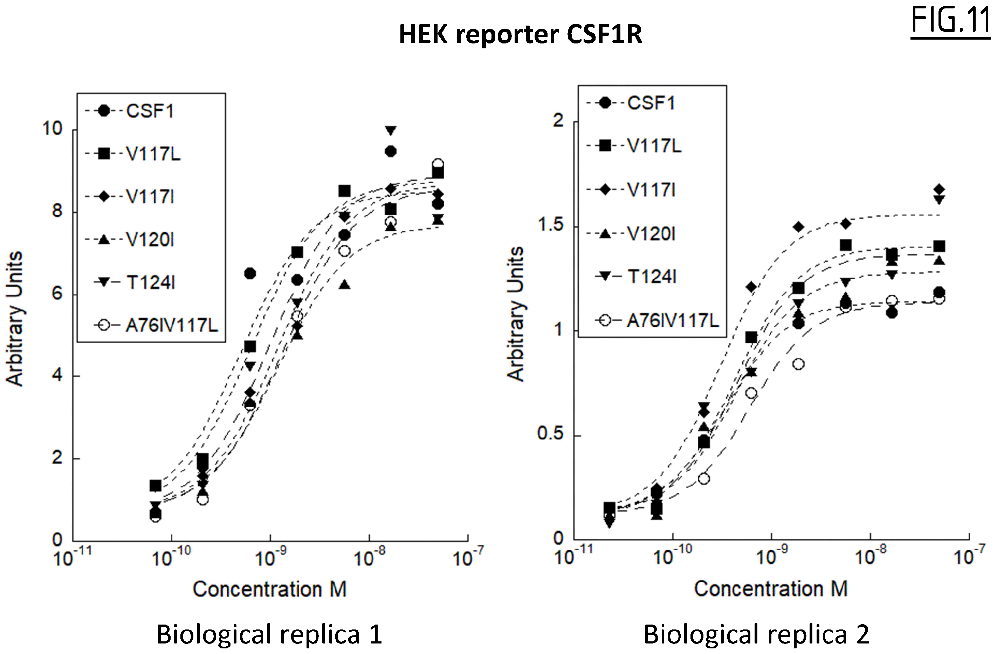

**Figure 11. Dose-response analysis of CSF1 (SEQ ID NO: 12) variants designed to enhance stability of CSF1.** The analysis was performed in HEK293 reporter cells and a curve-fit was inferred. The numbering of the mutants is based on SEQ ID NO: 12. The data has been fitted to a sigmoidal dose-response to determine the EC-50 (see Methods). We have fixed the basal values and the Hill slope. Hill slope was fixed to -1.7 and baseline to 0.3.

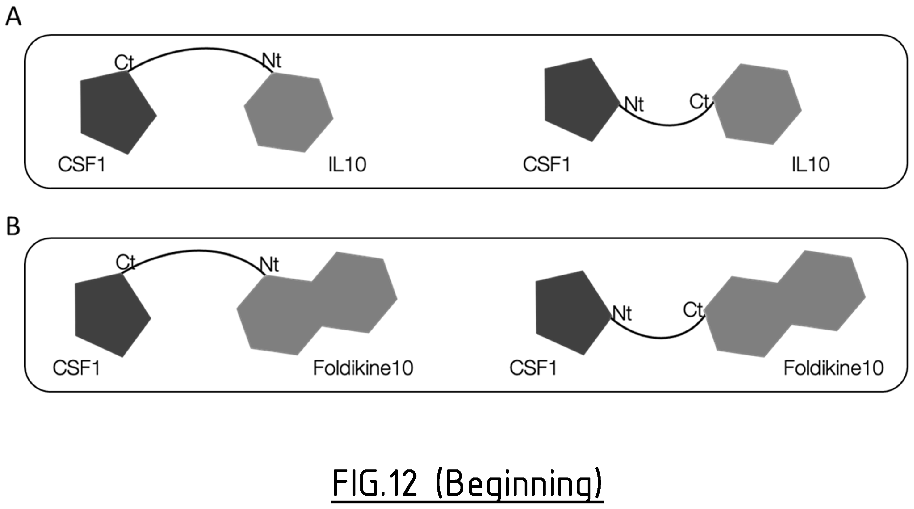

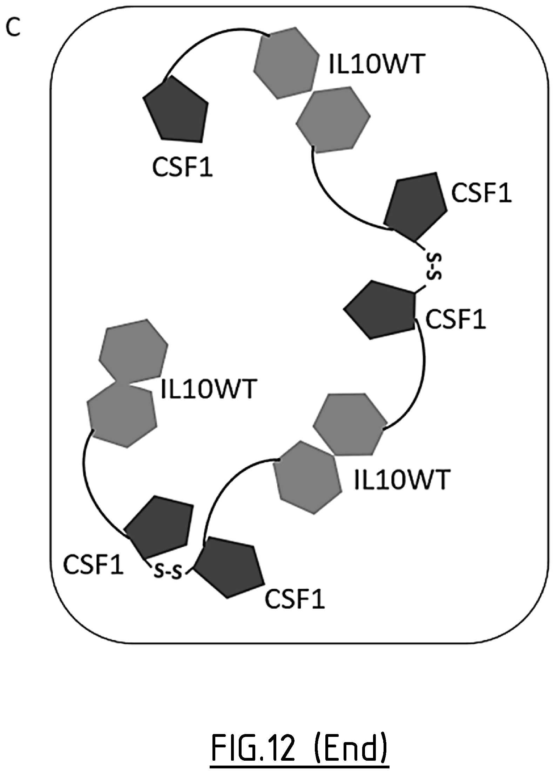

**Figure 12. Diagram displaying possible fusion proteins of IL-10 and CSF1 (A), as well as of Foldikine10 and CSF1 (B).** (C): display of how the IL-10 WT monomer fused to CSF1 might aggregate or generate concatemers.

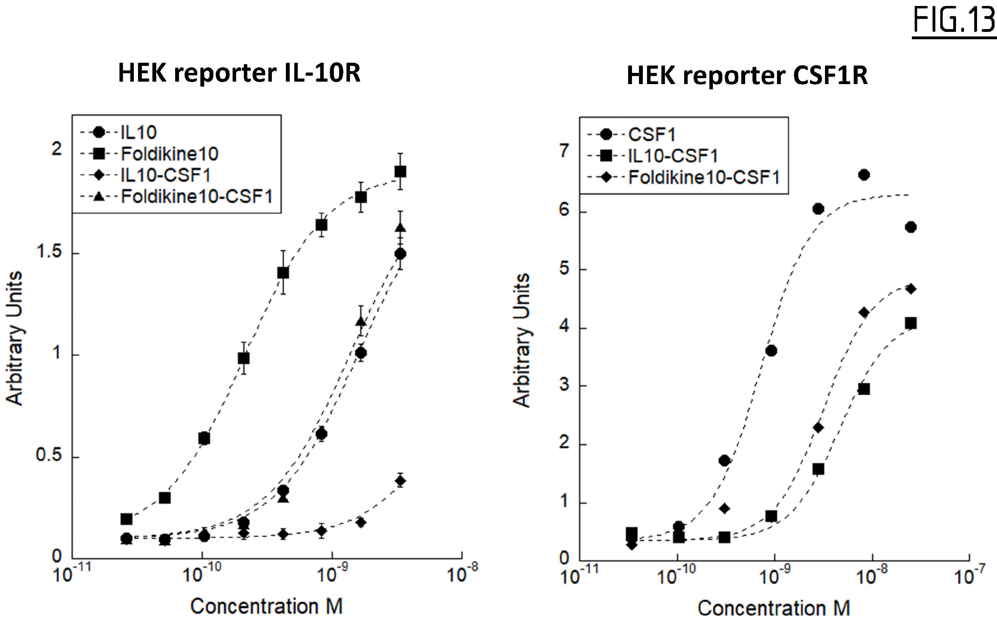

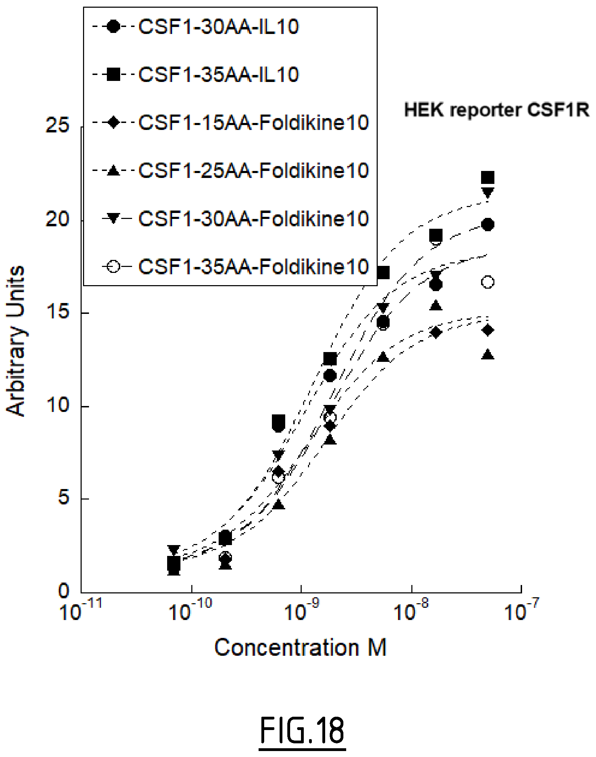

**Figure 13. Determination of EC-50 for the fusion of IL10 or Foldikine10 to CSF1 in Orientation A.** Titration curves of HEKblue cells IL10R (upper section) and CSF1R reporter cells (lower section) with increasing concentrations of candidate variants by keeping orientation A (IL-10 variant- linker- CSF variant). The length of the linker is the same as in Figure 18 (30 aa). CSF1 (REF) is SEQ ID NO:12. The data has been fitted to a sigmoidal dose-response to determine the EC-50 (see Methods). For the upper panel we have fixed the baseline to 0.08 and the Hill slope to -1.38. For the lower panel we have fixed the baseline to 0.3 and the Hill slope to -1.7. IL10 is IL10-HiBit (SEQ ID NO: 50). IL-10-CSF1 is IL-10-CSF1-HiBit (SEQ ID NO: 52), Foldikine10-CSF1 is Foldikine10-CSF1-HiBit (SEQ ID NO: 53).

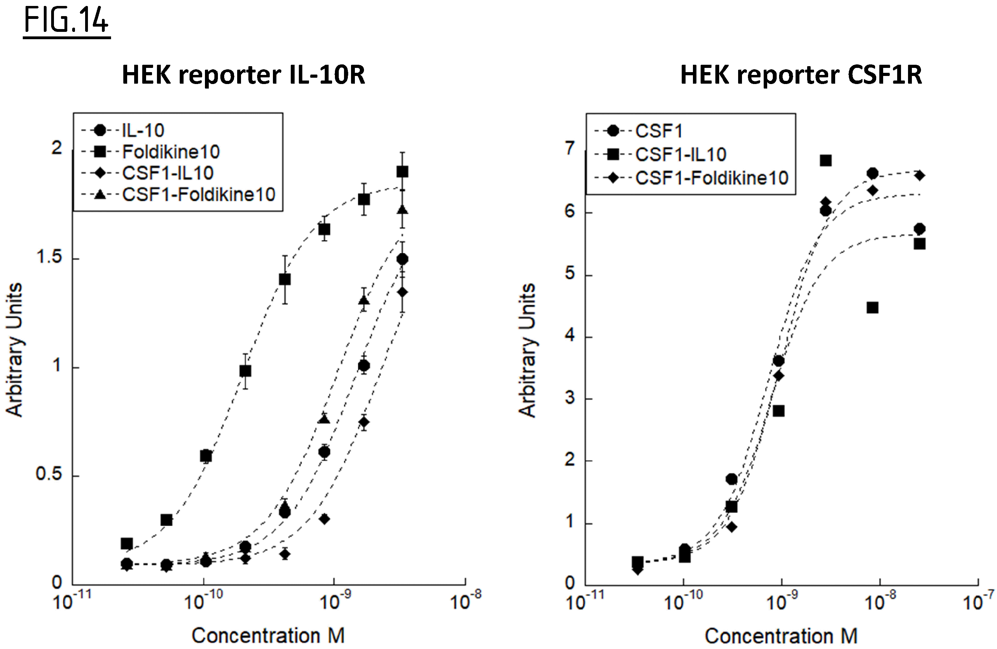

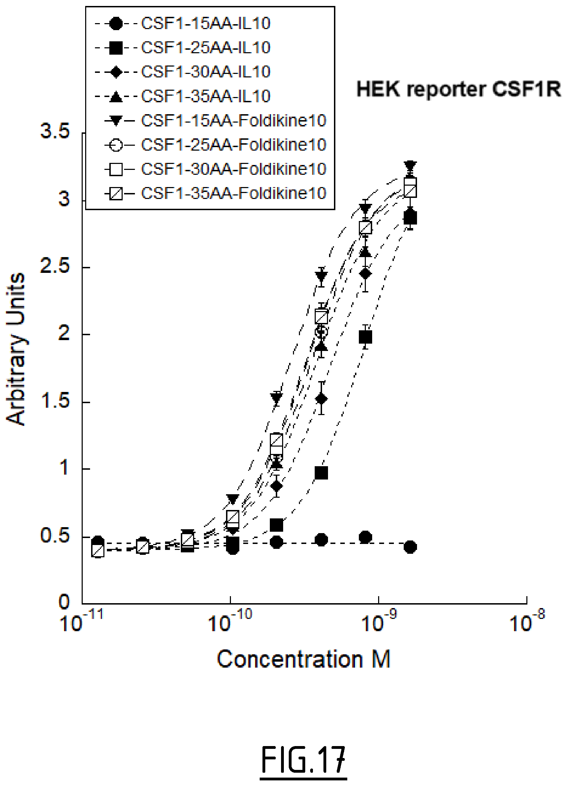

**Figure 14. Determination of EC-50 for the fusion of IL10 or Foldikine10 to CSF1 in Orientation B.** Titration curves of HEKblue cells IL10R reporter cells (left section) and CSF1R reporter cells (right section) with increasing concentrations of candidate variants by keeping orientation B (CSF variant- linker- IL10 variant). The length of the linker is the same as in Figure 17 (30 aa). CSF1 (REF) is SEQ ID NO: 12. The data has been fitted to a sigmoidal dose-response to determine the EC-50 (see Methods). For the upper panel we have fixed the baseline to 0.08 and the Hill slope to -1.38. For the lower panel we have fixed the baseline to 0.3 and the Hill slope to -1. IL10 is IL10-HiBit (SEQ ID NO: 50). CSF1-IL10 is CSF1-IL10-HiBit (SEQ ID NO: 54), CSF1-Foldikine10 is CSF1-Foldikine10-HiBit (SEQ ID NO: 55).

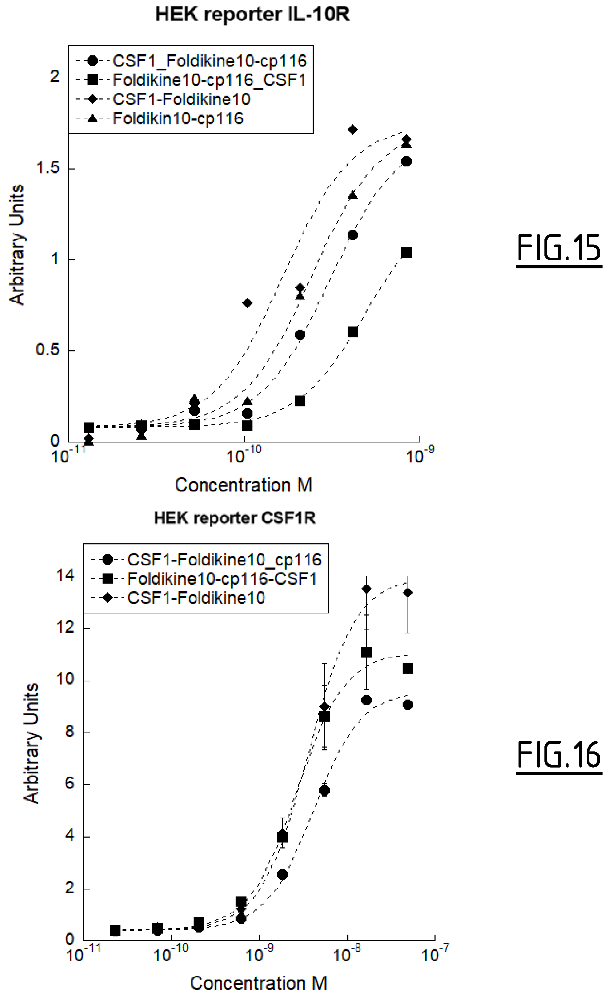

**Figure 15. Determination of EC-50 for the fusion of Foldikine10_cut116 to CSF1.** Titration curves for HEKBlue IL10R reporter cells of foldikine10_cut116 fused to CSF1. The data has been fitted to a sigmoidal dose-response to determine the EC-50 (see Methods). We have fixed the basal and the Hill slope values. CSF1-Foldikine10_cut116 is CSF1-Foldikine10_cut116-HiBit (SEQ ID NO: 57), Foldikine10_cut116-CSF1 is Foldikine10_cut116-CSF1-HiBit (SEQ ID NO: 56), CSF1-Foldikine10 is CSF1-Foldikine10-HiBit (SEQ ID NO: 55), foldikine10_cut116 is SEQ ID NO: 5.

**Figure 16. Determination of EC-50 for the fusion of Foldikine10_cut116 to CSF1.** Dose-response curve of HEK transformed with CSF1R reporter kit. The data has been fitted to a sigmoidal dose-response to determine the EC-50 (see Methods). We have fixed the basal and the Hill slope values. CSF1-Foldikine10_cut116 is CSF1-Foldikine10_cut116-HiBit (SEQ ID NO: 57), Foldikine10_cut116-CSF1 is Foldikine10_cut116-CSF1-HiBit (SEQ ID NO: 56), CSF1-Foldikine10 is CSF1-Foldikine10-HiBit (SEQ ID NO: 55).

**Figure 17. Linker length and protein functionality for orientation B.** Data for HEKBlue IL-10 R reporter cells. The data has been fitted to a sigmoidal dose-response to determine the EC-50 (see Methods). We have fixed the basal and the Hill slope values. CSF1-25aa-foldikine10 is SEQ ID NO: 58, CSF1-30aa-foldikine10 is SEQ ID NO: 55, CSF1-35aa-foldikine10 is SEQ ID NO: 59, CSF1-15aa-foldikine10 is SEQ ID NO: 60, CSF1-15aa-IL10 is SEQ ID NO: 61, CSF1-25aa-IL10 is SEQ ID NO: 62, CSF1-30aa-IL10 is SEQ ID NO: 63, CSF1-35aa-IL10 is SEQ ID NO: 64. The Hill slope was fixed to -1.2 and the baseline to 0.40.

**Figure 18. Linker length and protein functionality for orientation B.** Data HEK CSF1R reporter cells. The protein concentration for IL-10 variants with 15 and 25 amino acid (aa) linker was lower than the dynamic range of HEK CSF1R reporter cells. CSF1-25aa-foldikine10 is SEQ ID NO: 58, CSF1-30aa-foldikine10 is SEQ ID NO: 55, CSF1-35aa-foldikine10 is SEQ ID NO: 59, CSF1-15aa-foldikine10 is SEQ ID NO: 60, CSF1-15aa-IL10 is SEQ ID NO: 61, CSF1-25aa-IL10 is SEQ ID NO: 62, CSF1-30aa-IL10 is SEQ ID NO: 63, CSF1-35aa-IL10 is SEQ ID NO: 64. The protein expression for two mutants (CSF1-15AA-IL10 and CSF1-25AA-IL10 was below the dynamic range of the CSFF1R HEK reporter cells). The Hill slope was fixed to -1.06 and the baseline to 1.2.

**Figure 19. Schematic diagram showing the possible masking effect due to the way the fused CSF1 protein is connected to the Foldikine10 or the circular permutants of Foldikine 10.** A) Schematic diagram showing the binding of Foldikine 10 to its two receptors where the natural Nt and Ct point to the cell membrane. B) Schematic diagram showing the binding of the fusion of the natural Nt of Foldikine 10 to the Ct of CSF1 (The same will happen if fused to the Ct of Foldikine10). In this case the presence of CSF1 fused to the Nt will affect the binding of the left receptor causing a masking effect and decreasing Foldikine10 binding affinity. C) Schematic diagram showing the binding of the fusion a new Nter creating by opening a loop that points away from the membrane to a circular permutant of Foldikine 10 to the Ct of CSF1 (The same will happen if fused to the Ct of the circular permutant). In this case the presence of CSF1 fused to the Nt will not affect the binding of the left receptor.

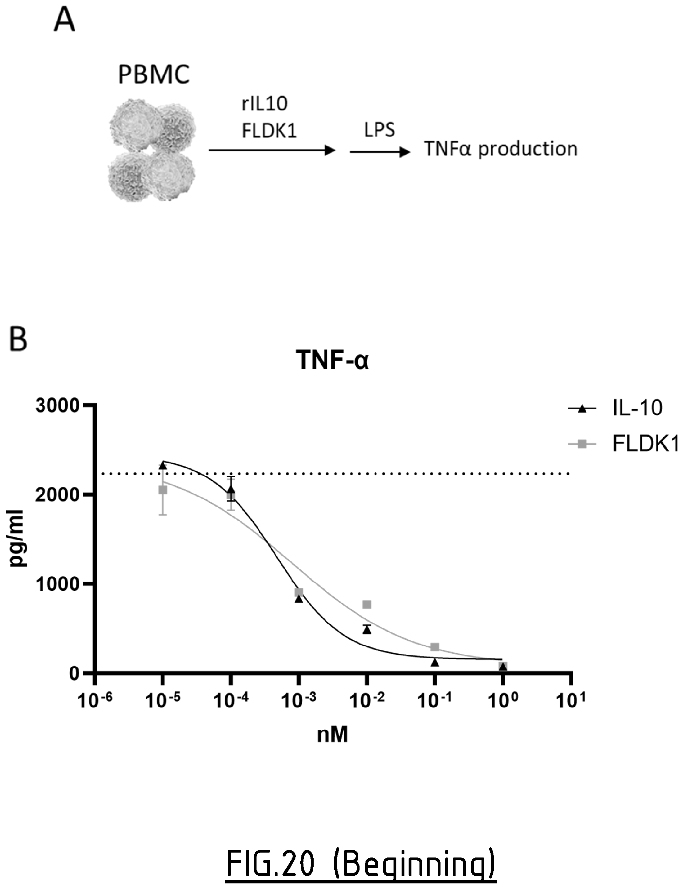

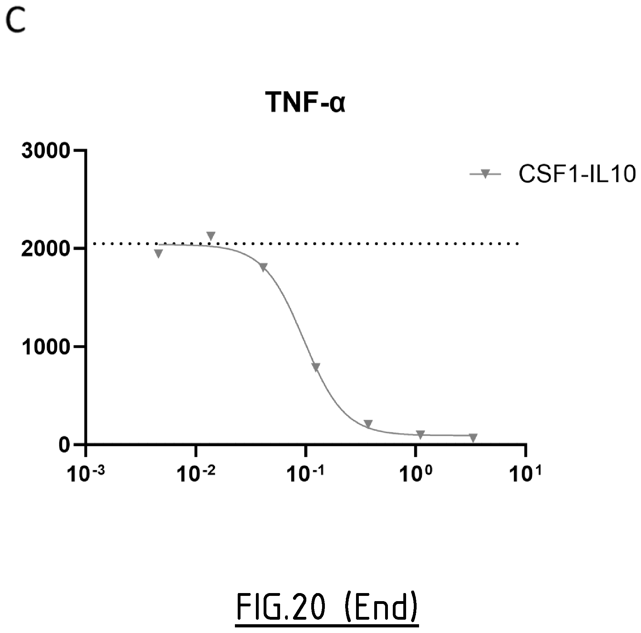

**Figure 20. FLDK-1 and CSF1-IL10, similarly to IL-10, inhibit monocytes activation.** (A) Total Peripheral blood mononuclear cells (PBMC, as abbreviated in English) from healthy donors were stimulated with LPS in the presence or absence of different concentrations of IL-10, FLDK-1 or CSF1-IL10. (B) TNF$\alpha$ levels in the supernatants of PBMCs treated with rIL10 or FLDK1 were determined by ELISA 24h after stimulation. (C) TNF$\alpha$ levels in the supernatants of PBMCs treated with CSF1-IL10 were determined by ELISA 17h after stimulation.

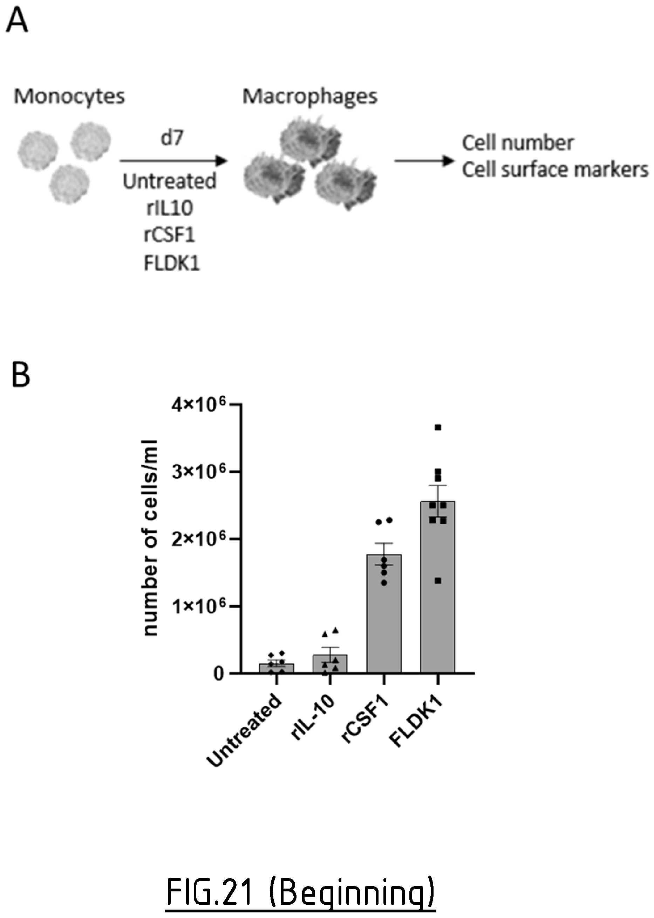

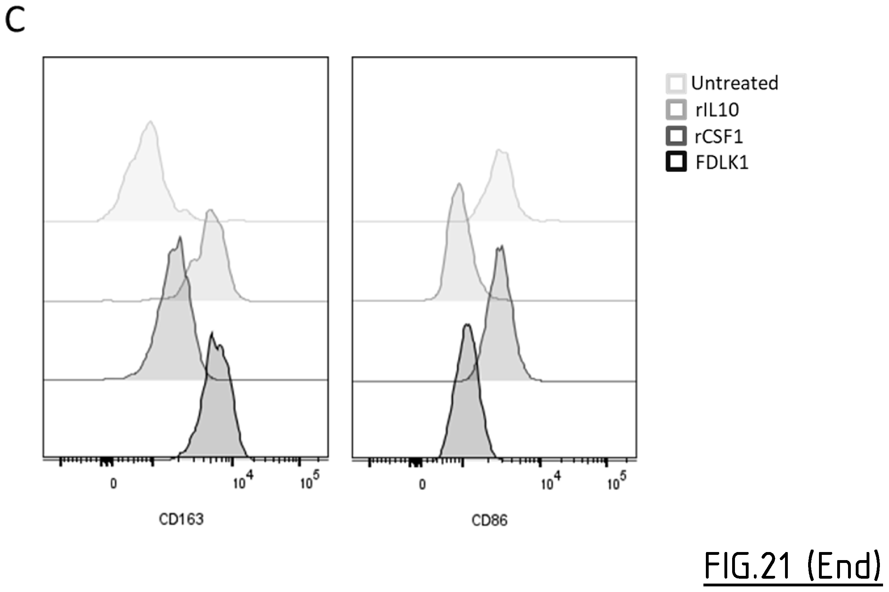

**Figure 21. FLDK1, similarly to CSF1, induces the differentiation of monocyte-derived macrophages.** (A) PBMCs were isolated from human buffy coats and CD14+ cells were purified by positive microbead selection. Monocytes were cultured for 7 days in the presence or absence of: rIL-10, rCSF1, FLDK1 or untreated condition. Cytokines were added every 2 days. (B-C) At day 7 alive cells were counted (B) and the levels of expression of macrophage markers (CD14, MHCII, CD206, CD163, and CD86) determined by FACS (Flow cytometry) represented only CD163 and CD86 (C). Data shown as a group of between 4-8 different experiments as mean + SEM.

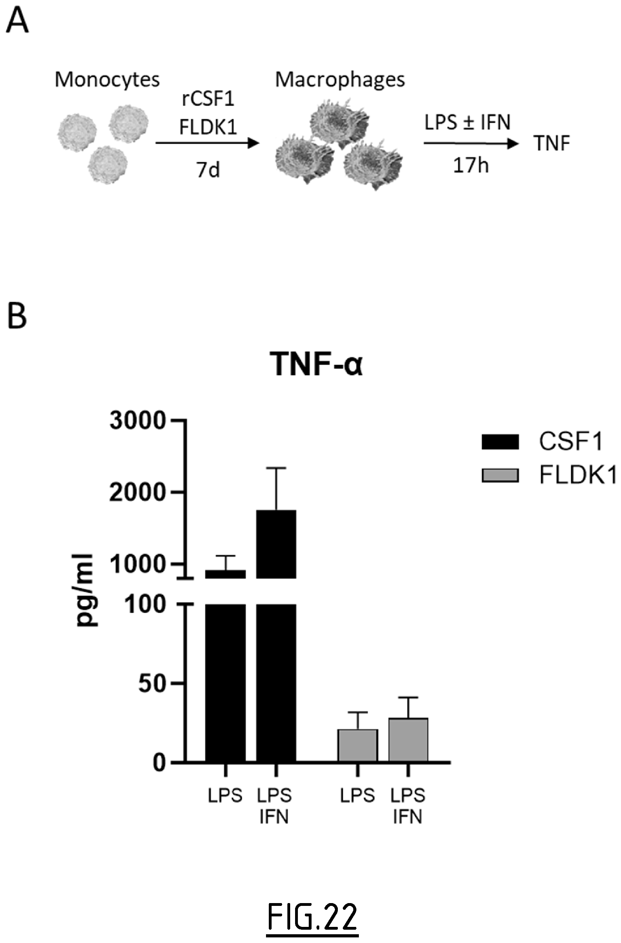

**Figure 22. FLDK1-induced macrophages have a regulatory phenotype.** (A) PBMCs were isolated from human buffy coats and CD14+ cells were purified by positive selection. Monocytes were cultured for 7 days in the presence or absence of: rCSF1 or FLDK1. Cytokines were added every 2 days. At day 7, cells were harvested and stimulated with LPS±IFN$\gamma$. (B) Cytokines levels in the supernatants were determined by ELISA 17h after stimulation. Data shown as a group of between 3-4 different experiments as mean + SEM.

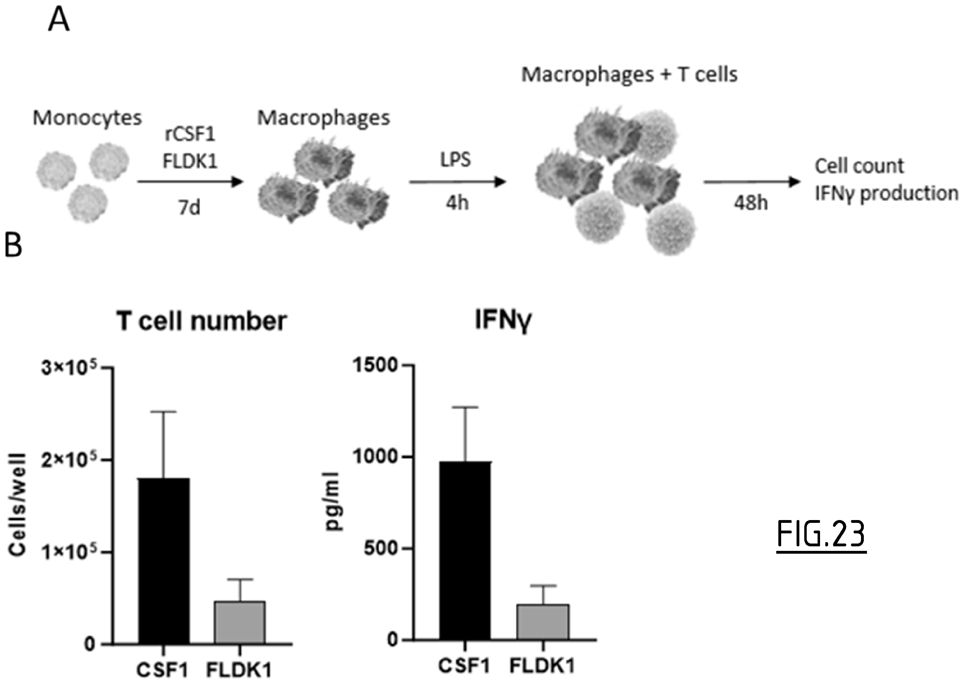

**Figure 23. FLDK1-induced macrophages have a regulatory phenotype.** (A) Monocytes were isolated from human buffy coat samples by selecting for positive CD14 cells. Monocytes were cultured for 7 days in the presence or absence of: rCSF1 or FLDK1. Cytokines were added every 2 days. At day 7 cells were detached from plates and counted, the same number of macrophages were replated and stimulated with LPS for 4 hours. CD3+ cells from a different donor were purified, counted and added to CSF1 or FLDK1-differentiated macrophages. Cytostim (Miltenyi) was also added to the co-cultures to enhance the interaction between both cell types. At 48h supernatants were harvested and cells were counted after 5 days in co-culture. (B) Data shown as a pool between 4-6 different experiments as mean + SEM.

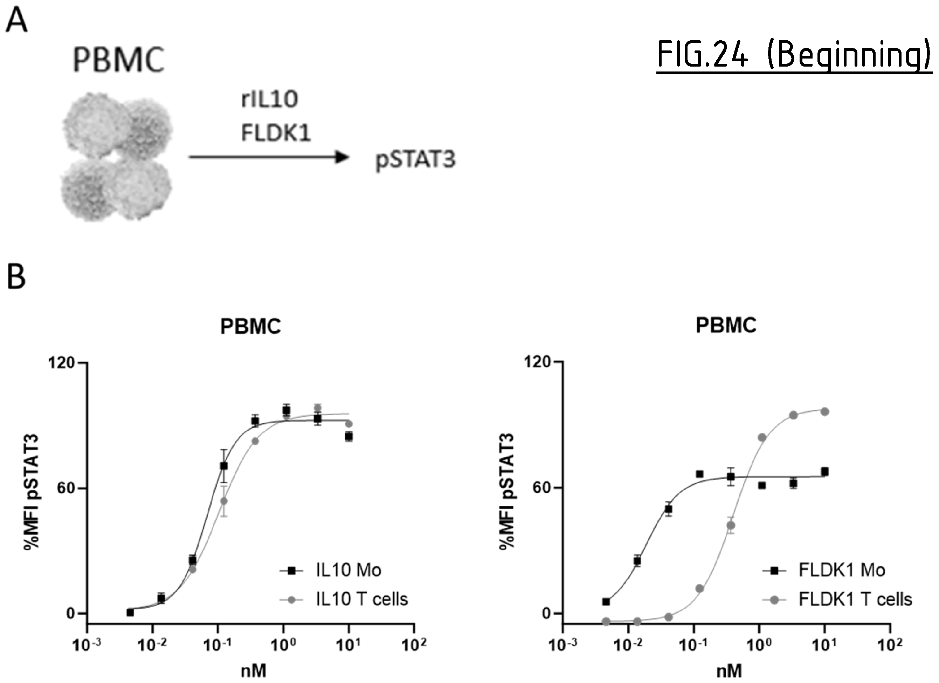

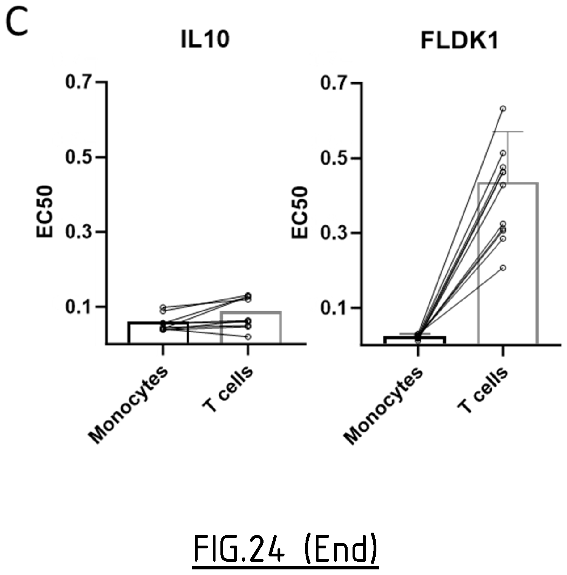

**Figure 24. FLDK-1 shows selectivity towards myeloid cells compared to lymphoid cells.** (A) PBMC from healthy donors were plated on in 96-well plates and stimulated next day with rIL10 or FLDK1 for 20 min at 37°C. Cells were stained with CD14 Alexa647 and CD3 APC/Cy7, fixed with 4% PFA and permeabilized for intracellular staining with 80% MetOH. Cells were stained with AlexaFluor488 pSTAT3 (pY705). (B-C) Samples were analyzed with LSRII cytometer and MFI *("Mean Fluorescence Intensity")* values obtained from STAT3 activation were plotted using GraphPad Prism software. One representative result with two technical replicates is shown as mean with error bar depicting the SEM. Each biological replicate was normalized by assigning the highest IL10 MFI value of the top concentration as 100% and the lowest MFI value of an untreated control as 0%. The MFI from the samples treated with FLDK1 was normalized accordingly.

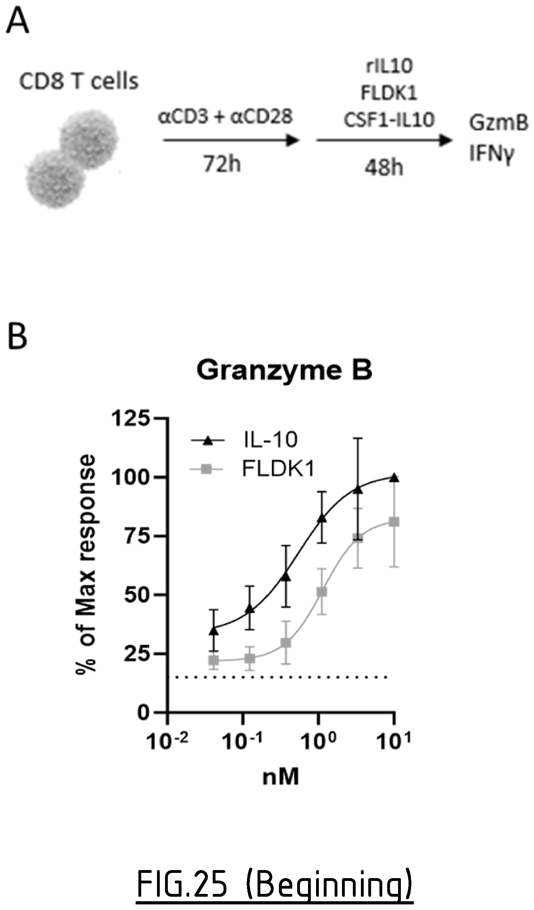

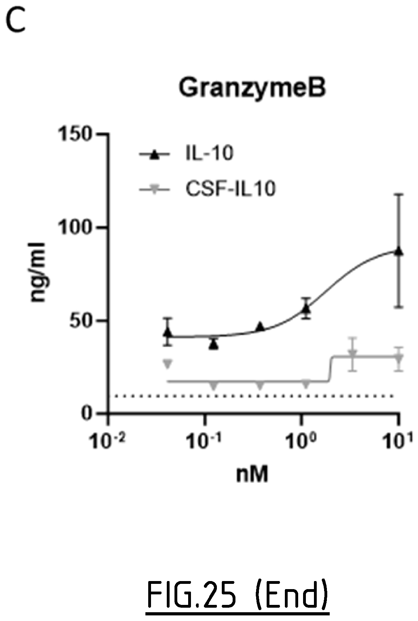

**Figure 25. FLDK-1 and CSF1-IL10 are less potent than IL-10 at activating CD8 T cells.** (A) CD8 T cells were isolated from human PBMCs using positive microbeads selection (Miltenyi). The lymphocytes were stimulated with anti-CD3 and anti-CD28 for 72 hours. Then, cells were splited in two and treated with (B) IL10 or FLDK1 or (C) IL10 or CSF1-IL10 with IL-2 for 48 hours. Cell supernatants were obtained and Gzmb and IFN$\gamma$ were detected by ELISA. Pooled normalized data of 4 independent experiment is shown in the graph. t-test IL-10 vs FLDK1 p-value < 0.05.

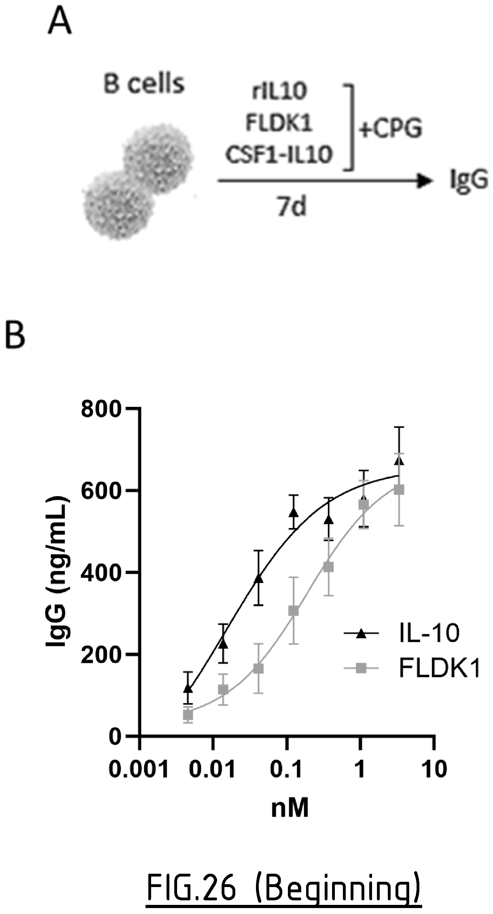

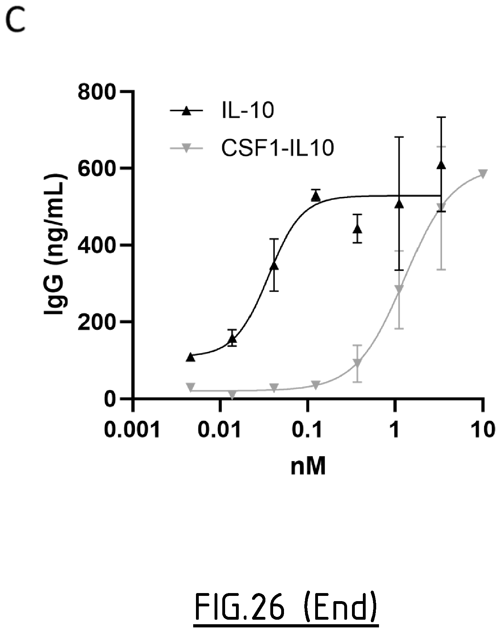

**Figure 26. FLDK-1 and CSF1-IL10 are less potent than IL-10 at activating B cells.** (A) B cells were isolated from human PBMCs using positive microbeads selection. The isolated lymphocytes were stimulated with CpG (InvivoGen) and (B) IL10 or FLDK1, or (C) CSF1-IL10 or IL10, at days 0 and 2. After 7 days the levels of IgG in the supernatants were determined by ELISA. Pooled data of 4 independent experiment is shown in the graph. t-test IL-10 vs FLDK1 p-value < 0.05.

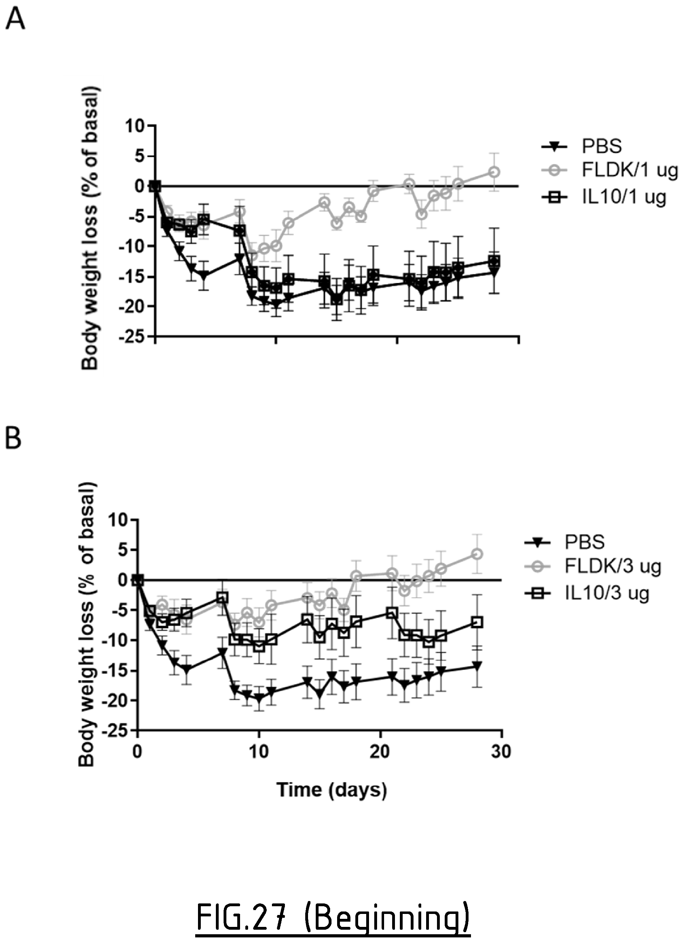

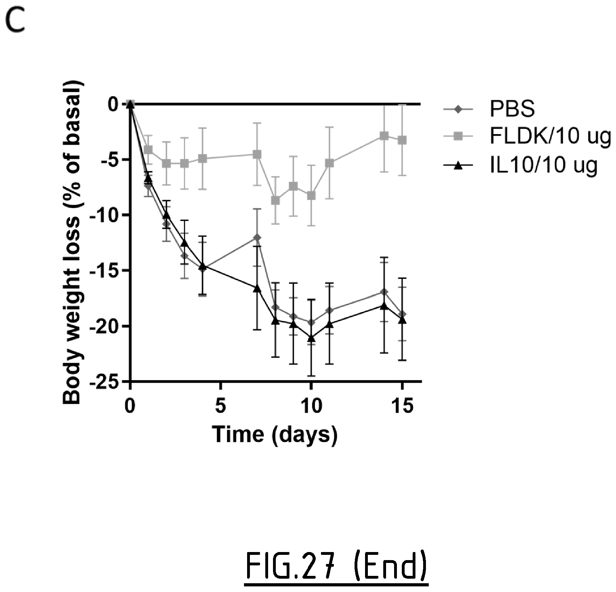

**Figure 27. FLDK1 protects mice from chronic TNBS-induced colitis.** Evaluation of therapeutic efficacy of FLDK1, compared to IL-10, (in three doses 1$\mu$g per mice, 3$\mu$g per mice or 10 $\mu$g per mice) in an experimental model of chronic IBD induced by TNBS. Mice treated with FLDK1 had less body weight loss compared to both IL10 treated or vehicle treated after 30 days treatment.

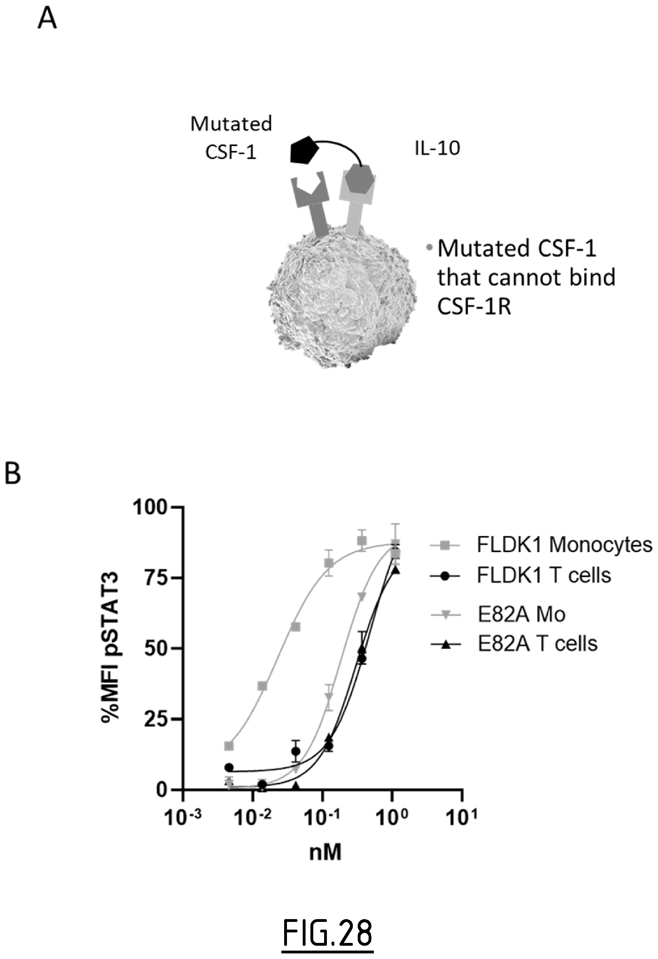

**Figure 28. Structure-Activity Relationship.** (A) PBMC from healthy donors were plated on 96-well plates and stimulated next day with rIL10, FLDK1, or FLDK1-E82A. (B) Samples were analyzed with LSRII cytometer and MFI values obtained from STAT3 activation were plotted using GraphPad Prism software.

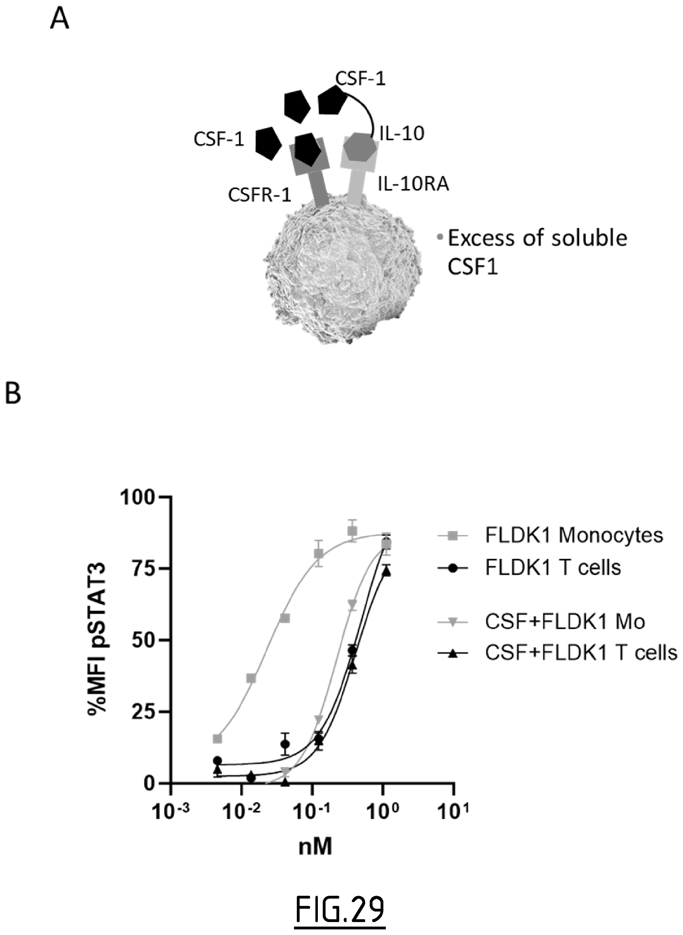

**Figure 29. Structure-Activity Relationship.** (A) PBMC from healthy donors were plated on 96-well plates and stimulated next day with rIL10, FLDK1, or saturating concentrations of CSF1 + FLDK1. (B) Samples were analyzed with LSRII cytometer and MFI values obtained from STAT3 activation were plotted using GraphPad Prism software.

## EXAMPLES

### Example 1: Production of CSF1-IL10 combined bioactivity in a single-chain protein

Our goal is to create a dual molecule that can activate IL-10 signaling exclusively in cells expressing CSF1R, which are predominantly myeloid cells and no other cells such as CD8+ T cells. To achieve this, we have engineered variants of IL-10 and/or Foldikine-10 with reduced affinity to IL10RA and IL10RB linked to a CSF1 monomer. The CSF1 component acts as a targeting tool to circulating monocytes, facilitating preferential binding of IL-10 to these cells (see Figure 1).

*Engineering the Foldikine10 chassis*

The proposed product/s, Folkidine™-1 involves variants of IL-10 and CSF1 linked at a specific distance and orientation. To engineer the IL-10 variant, we have designed Nter-Cter closing linkers (intralinker) in the IL-10 swapped domain to end up with a single chain (Foldikine™ scaffold, Figure 2) (Foldikine10, based on the so-called MutSC1 construct in patent application PCT/EP2023/052202 with D>E substitution at position 79 of MutSC1; Montero-Blay et al., Molecular Systems Biology (2023)19:e11037), and molecules ORK10-002, ORK10-003, ORK10-005).

Foldikine10 (SEQ ID NO: 1) comprises amino acids at positions 5 to 157 of IL-10 (SEQ ID NO: 11) with the substitution G5M, D84E and K157N (SEQ ID NO: 28), a Nt-Ct linker peptide consisting of NGGLDY (SEQ ID NO: 30), and amino acids at positions 19 to 160 of IL-10 (i.e. SEQ ID NO: 29). SEQ ID NO: 20 (foldikine10-HiBit) is the same as SEQ ID NO: 1 except for the addition at the C-terminus of a HiBit tag for protein quantification.

ORK10-002 (SEQ ID NO: 2) differs from Foldikine10 (SEQ ID NO: 1) by substitution of N154F and the reverse substitution E80D (i.e. ORK10-002 includes the Asp (D) residue present at position 84 of IL-10 as shown in sequence SEQ ID NO:11). ORK10-002 comprises amino acids at positions 5 to 152 of IL-10 with the substitution G5M (SEQ ID NO: 39), a Nt-Ct linker peptide consisting of FGGLDY (SEQ ID NO: 46), and amino acids at positions 19 to 156 of IL-10 (of sequence SEQ ID NO: 40).

ORK10-003 and ORK10-003-HiBit (SEQ ID NO: 3 and SEQ ID NO: 49) and ORK10-005 (SEQ ID NO: 4) also comprise the reverse substitution E80D compared to Foldikine10 (SEQ ID NO: 1) and further differ by engineering of the Nt-Ct linking and surrounding residues of the IL-10 monomers.

ORK10-003 (SEQ ID NO: 3) comprises amino acids at positions 5 to 154 of IL-10 (SEQ ID NO: 11) with the substitutions G5M (SEQ ID NO: 39) and M154 mutated to L (SEQ ID NO: 41), a Nt-Ct linker peptide consisting of YKTIT (SEQ ID NO: 31), and amino acids at positions 17 to 156 of IL-10 (SEQ ID NO: 424) where residues GNLP are mutated to PEFA. SEQ ID NO: 49 (ORK10-003-HiBit) is the same as SEQ ID NO: 3 except by the addition at the C-terminus of a HiBit tag for protein quantification.

ORK10-005 (SEQ ID NO: 4) comprises amino acids at positions 5 to 153 of IL-10 (of sequence SEQ ID NO: 43), a Nt-Ct linker peptide consisting of DKDIRDGD (SEQ ID NO: 32), and amino acids at positions 18 to 156 of IL-10 (of sequence SEQ ID NO: 44).

Comparison of the EC-50 for Foldikine10 (SEQ ID NO: 1), and ORK10-002 (SEQ ID NO: 2), ORK10-003 (SEQ ID NO: 3), and ORK10-005 (SEQ ID NO: 4) shows that molecule ORK10-003 has an improved EC-50 when compared to Foldikine-10 (Figure 3, Table 1).

Table 1. EC-50 values and its associated errors for the different IL-10 foldikine variants

|  | EC-50 | error EC-50 |
|---|---|---|
| IL-10 | 8.91E-10 | 4.24E-11 |
| Foldikine10 | 6.01E-10 | 4.22E-11 |
| ORK10-002 | 3.87E-10 | 2.70E-11 |
| ORK10-003 | 2.30E-10 | 2.95E-11 |
| ORK10-005 | 6.25E-10 | 2.69E-10 |

Baseline fixed to 0.27 and Hill slope fixed to -1.6 for all variants except ORK10-005 that has a clear different slope.

*Circular permutants of Foldikine 10.*

The natural Nt and Ct of Foldikine10 and the different ORK-10s are pointing to the cell membrane when the cytokine molecules are bound to the IL10RA and IL10RB receptors (Figure 4). This could result in a masking effect when fusing it to another cytokine like CSF1 due to steric hindrance of the fused molecule with the cell membrane or the receptors. To alleviate this effect, we have done circular permutants of Foldikine10 by exploring the structure opening over all flexible regions connecting $\alpha$-helices (thus creating non-natural Nt and Ct) while linking the free Nt and Ct with the same linker used to engineer Foldikine10 (Sequences Foldikine10_cut116 (SEQ ID NO: 5), Foldikine10_cut133 (SEQ ID NO: 6), Foldikine10_cut209 (SEQ ID NO: 7), Foldikine10_cut237 (SEQ ID NO: 8)). We first expressed the different versions and quantified by ELISA. For only Foldikine10_cut116 we had enough protein to test in HEK reporter cells. This molecule (Foldikine10_cut116) was active (cp116) but decreased EC-50 (EC50 in HEK reporter IL-10 is 6.04E-10 for Foldikine10, and 1.67E-09 for Foldikine10_cut116). This molecule involves opening one of the regions connecting the two 3D IL-10 domains at position 116 (see Figure 4). Cutting at this point should allow connecting to other molecules without causing a masking effect (see section below) since the new Nt and Ct are pointing opposite to the membrane and far away from the receptors (see Figure 3).

*CSF1 structure and circular permutants of CSF1*

CSF1 naturally dimerises with the two monomeric molecules face to face and hold together by a disulphide bond (see Figure 4).

Circular permutants of CSF1 were generated, where the native Nt-Cts are connected by an engineered linker and the novels Nt- and Cts are generated by disconnecting the peptide bond in a region of the protein that does not disrupt the interaction with the receptor (Between residues 95 and 100, Figure 6). Both novel molecules (ORKMCSF_013 (SEQ ID NO: 9) and ORKMCSF_014 (SEQ ID NO: 10); Figure 7) were capable to activate the downstream CSF1 signaling cascade (Figure 8) with similar EC-50 values and slightly better than the monomeric CSF1 molecules (Table 2).

Table 2. EC-50 values and its associated errors for the different CSF1 variants

|  | EC-50 | error EC-50 |
| --- | --- | --- |
| CSF1 (1-190) HiBiT | 1.45E-09 | 3.00E-10 |
| ORKMCSF_013 | 7.37E-10 | 9.49E-11 |
| ORKMCSF_014 | 7.71E-10 | 1.74E-10 |

**Engineering IL-10 affinity**

We engineered a set of mutants based on the ORK10-003-HiBit foldikine10 active scaffold (SEQ ID NO: 49) to decrease IL10RA affinity. We explored 11 mutants using FoldX and sequence conservation analysis, through which we identified a series of mutations that did not compromise protein expression and reduced the EC-50 (R24A, E50A, D44N, K34A, Q38A, D44A, numbering based on >SEQ ID NO: 11b IL10b, see Methods). R24 is R163, K34 is 173, Q38 is 177, D44 is 183, E50 is 189 in ORK10-003-HiBit. These mutations are point mutations integrated on the continuous domain of Foldikine10. These mutants exhibited different degree of activation of the HEKblue cell reporters with EC-50 (see Figure 8, Table 3). A mutation that weakened IL10RB interaction was also identified (N92E ; numbering based on >SEQ ID NO: 11 IL10, N92 is 231, in ORK10-003-HiBit) (see Figure 9, Table 3).

Table 3. EC-50 values and its associated errors

|  | EC-50 | error EC-50 |
| --- | --- | --- |
| Foldikine10 (ORK10-003 REF) | 9.44E-11 | 4.12E-12 |
| R24A | 1.51E-10 | 1.16E-11 |
| E50A | 5.43E-11 | 3.40E-11 |
| D44N | 4.37E-10 | 1.84-E11 |
| D44A | 2.01E-9 | 2.57E-11 |
| K34A | 9.22E-10 | 5.51E-12 |
| Q38A | 2.07E-10 | 6.38E-11 |
| Foldikine10 (ORK10-003 REF) | 9.53E-11 | 4.14E-12 |
| N92E | 1.19E-10 | 3.84E-12 |

We fixed the basal line in the fitting to -0.27 and the Hill slope to -1.6 for the first 7 rows and to 0.12 and -1.9 for the last two rows.

**Mutants to increase EC-50 of CSF1 with CSF1 receptor**

For CSF1, we engineered a set of single- or double-point mutations to increase affinity for CSF1R (Table 4). We selected a set of conserved and non-conserved residues based on the changes in complex interaction energy with the receptor and in stability of the mutated CSF1 predicted by FoldX, and visual inspection of the complex interface (in bold the residues conserved (allowing one mismatch) between the CSF1 sequences of mouse, Hamster, Human, Pig, Cow, Sheep, Alpaca, Camel, Fox, Dog, Vison, Wallrus, Otter, Polar bear, Cat, Puma, Dolphin, Bat, Raccoon, Blue Whale and Horse; with an asterisk, the residues mutated for improving binding and with 'x' those mutated to increase stability of CSF1).

```
E EVSEYCSHMIGSGHLQSLQRLIDSQMETSCQITFEFVDQEQLKDPVCYLKKAFLLVQDIMEDTMRFR
        x*        xx                        *
DNTPNAIAIVQLQELSLRLKSCFTKDYEEHDKACVRTFYETPLQLLEKVKNVFNETKNLLDKDWNIFS
        * xx xx            *                         *   *    *
KNCNNSFAECSSQD  (SEQ ID NO:12).
```

**Table 4.** Selected CSF1 mutants including the predicted FoldX energies modeled on CSF1/CSF1R complex (should be noted that FoldX has an error of around 0.8 kcal/mol in its predictions).

| Mutations | Δ Interaction Energy (kcal/mol) | Δ Complex Stability (kcal/mol) | Δ VdW clashes (kcal/mol) |
|---|---|---|---|
| H9F | -1.34 | -0.80 | -0.03 |
| H9L | -0.64 | -0.89 | -0.11 |
| H9Q | -0.39 | -0.56 | -0.04 |
| Q17K | -0.39 | 0.00 | -0.01 |
| Q17R | -0.88 | -0.48 | 0.08 |
| S18F | -0.35 | -0.67 | 0.06 |
| S18L | -0.16 | -0.87 | -0.02 |
| S18M | -0.33 | -0.73 | 0.01 |
| S18Q | 0.04 | 0.14 | -0.01 |
| V78F | -0.90 | -0.43 | 0.00 |
| V78I | -0.16 | -0.61 | 0.00 |
| V78L | -0.37 | -0.65 | 0.00 |
| V78M | -0.09 | -0.29 | 0.00 |
| V78Y | 0.38 | 0.00 | 0.03 |
| V78W | -0.74 | -0.65 | 0.04 |
| Q79L | -1.30 | -1.54 | -0.01 |
| Q81F | -1.43 | -1.76 | 0.00 |
| Q81Y | -1.80 | -2.71 | 0.06 |
| E82K | -1.89 | -1.80 | 0.08 |
| E82T | -0.89 | -0.79 | 0.33 |
| R86W | -0.37 | -0.65 | -0.05 |
| K93E | -0.37 | -0.40 | 0.00 |
| V117I | 0.000 | -1.60 | 0.00 |

For increased affinity, we selected mutations H9F, Q17R, S18Q, V78W, Q79L, Q81F, Q81Y, E82K. In a second round based on sequence conservation, we selected V78F, S18F-R21Q, Q17V/L, S18F, V78F/L/Y, Q79T/K, K93E, T93E, T34L and M10L.

In Figure 10, we show the titration curve of different CSF1 mutants and the EC-50 values and their associated errors. These variants were evaluated in HEK293 cells transfected with a CSF1R/reporter kit (see Methods). Of all the mutants two had similar EC-50 values as CSF1 WT (Q17R and V78W) been better in one of the two biological replicas.

**Table 5.** EC-50 values and its associated errors

| | CSF1 | H9F | Q17R | S18Q | V78W | Q79L | Q81F | E82K |
|---|---|---|---|---|---|---|---|---|
| **EC50 Biological replica 1** | 1.88E-09 | 2.66E-9 | 2.70E-9 | 2.51E-9 | 6.23E-9 | 6.76E-9 | 1.08E-8 | 1.27E-8 |
| **Error** | 8.63E-11 | 9.5E-10 | 5.31E-10 | 9.61E-10 | 3.57E-10 | 9.27E-10 | 1.45E-9 | 3.71E-9 |
| Hill slope fixed to -1.7 and basal line to 0.3 | | | | | | | | |
| **EC50 Biological replica 2** | 5.58E-09 | 1.18E-08 | 7.94E-09 | 1.99E-08 | 2.10E-08 | 1.28E-08 | 6.77E-08 | 2.49E-08 |
| **Error** | 1.09E-09 | 1.04E-09 | 9.38E-10 | 1.85E-09 | 3.96E-09 | 1.17E-09 | 5.34E-09 | 3.45E-09 |

Hill slope fixed to -1.7 and basal line to 0.45

In parallel, as discussed above, we designed different variants to increase the stability of CSF1 conformation in the bound state to CSF1R. These variants were evaluated in HEK293 cells transfected with a CSF1 R/reporter kit (see Methods) (See Table 6). The mutants were selected so as to involve residues not directly interacting with the CSFR1 receptor as well as being partly or totally buried in the structure. Of the mutants, we could see a slight increase in binding affinity for mutant V117L in one of the biological replicas and similar values as the WT for T124I in both biological replicas. More interestingly we found an increase in the maximum activation value in the HEK293 cells transfected with a CSF1R/reporter kit in both replicas but only significant in the second replica.

**Table 6.** The table shows the EC_50 and maximum response value determined in HEK cells transformed with the CSFR1 reporter kit.

| **EC-50 Biological replica 1** | CSF1 | V117L | V117I | V120I | T124I | A76I, V117L |
|---|---|---|---|---|---|---|
| EC-50 | 4.52E-10 | 1.04E-09 | 1.00E-09 | 8.80E-10 | 5.74E-10 | 1.18E-09 |
| Error | 1.55E-10 | 1.36E-10 | 1.52E-10 | 2.48E-10 | 6.87E-11 | 1.91E-10 |
| Max activation value | 8.49 | 8.66 | 7.64 | 8.86 | 8.73 | 8.55 |
| Error | 0.59 | 0.26 | 0.26 | 0.55 | 0.22 | 0.31 |
| Hill slope fixed to -1.2 and Baseline to 0.33 | | | | | | |
| **EC-50 Biological replica 2** | CSF1 | V117L | V117I | V120I | T124I | A76I, V117L |
| EC-50 | 3.37E-10 | 3.07E-10 | 4.37E-10 | 4.43E-10 | 4.21E-10 | 5.97E-10 |
| Error | 3.14E-11 | 5.36E-11 | 7.68E-11 | 1.39E-11 | 3.79E-10 | 9.27E-11 |
| Max activation value | 1.14 | 1.56 | 1.28 | 1.37 | 1.40 | 1.14 |
| Error | 0.017 | 0.05 | 0.04 | 0.08 | 0.02 | 0.04 |

Hill slope fixed to -1.33 and Baseline to 0.12

Based on this analysis we propose combinations of mutants that within the error could improve binding affinity (Q78R, V78W, V120I) and/or signaling range (V117L, V120I, T124I) of CSF1 for its receptor.

    i) Q17R, T124I and V1201
    ii) V78W, T124I and V1201
    iii) Q17R, V78W, T124I and V120I
    iv) V117L, T1241.

**Example 2: Generation of a dual CSF1-IL10 molecule**

***Determination** of the optimal orientation of the CSF1-IL10 product*

*Engineering of the chimeric Foldikine-10 with CSF1 or Foldikine-CSF1.*

To evaluate if by fusing the Foldikine-10 with CSF1, we still maintained the activity of the two cytokines we first tested the activity of IL10 using the HEKBlue 10 reporter and CSF1 activity using HEK293 transfected with CSF1R reporter kit. There are two possibilities to make the fusion protein, either we link the Ct of IL-10 or Foldikine10 to the Nt of CSF1, or we fuse the Nt of IL-10 or Foldikine10 to the Ct of CSF1 (and the same with the circular permutants of Foldikine10 and of CSF1) (See Figure 12). Fusion of IL-10 to CSF1 could result in the formation of concatemers since IL-10 is a swapped dimer and CSF1 dimerizes (See Figure 12). This will not be the case for the other fusions. Because of a CMC reason, for the protein fusion we decided to continue with foldikine-10 instead of ORK 10-003.

## Orientation A - IL10-CSF1

In this orientation, we fuse the Ct of IL-10, or Foldikine10, to the Nt of CSF1, resulting in IL-10-CSF1 (SEQ ID NO:52), or Foldikine10-CSF1 (SEQ ID NO: 53). The IL-10WT EC50 is affected when fused to CSF1 at the functionality level. Foldikine-10 EC-50 is also affected, but to a lesser extent than IL-10 WT. Regarding CSF1 functionality, it losses activity when fused to the C-terminus of IL-10 and less when fused to Foldikine-10 (see Figure 13 and Tables 7 and 8).

## Orientation B -CSF1-IL10

In this orientation, we fuse the Ct of CSF1 to the NT of IL-10, or Foldikine10, resulting in CSF1-IL-10 (SEQ ID NO: 54), or CSF1-Foldikine10 (SEQ ID NO: 55). The IL-10 WT and Foldikine10 EC50 is affected when fused to CSF1. Regarding CSF1 functionality, its losses activity when fused to IL-10 but not with Foldikine10 as in the case of orientation A (see Figure 14 and Tables 7 and 8).

**Table** 7. IL-10 EC-50 values as well the error of this EC-50.

|  | IL-10 | |
|---|---|---|
|  | **EC-50** | **error EC-50** |
| IL-10* | 8.00E-10 | 5.42E-11 |
| Foldikine10 | 2.14E-10 | 4.87E-12 |
| CSF-IL10 | 1.16E-9 | 2.31e-10 |
| CSF1-Foldikine10 | 1.06e-9 | 1.04e-10 |
| IL-10-CSF1 | 5.9 E-9 | 6.06E-10 |
| Foldikine10-CSF1 | 1.36E-9 | 1.02E-10 |
| Baseline fixed to 0.08, and Hill Slope to -1.38<br>*IL10 concentration of the active dimeric form estimated by Hibit is double than for Foldikine10, thus we divided the EC50 by two | | |

**Table 8.** CSF1 EC-50 values as well the error of this EC-50.

|  | CSF1 | |
|---|---|---|
|  | **EC-50** | **error EC-50** |
| CSF1 | 7.34E-10 | 1.06E-10 |
| CSF-1-IL10 | 7.81e-10 | 3.18e-10 |
| CSF1-Foldikine10 | 9.53e-10 | 7.37e-11 |
| IL10-CSF1 | 4.64E-09 | 5.04E-10 |
| Foldikine10-CSF1 | 3.18E-09 | 4.06E-10 |
| Baseline fixed to 0.3 and Hill Slope to -1.7 | | |

***Testing the activity of the CSF1 fused to a circular permutant of Foldikine10***

As described above, the fusion of Foldikine10 to CSF1 at its N-terminus or C-terminus increases the EC-50 of Foldikine10. This could be due as explained above because the natural Nt and Ct of Foldikine10 point towards the cell membrane in the complex with receptors IL10RA and IL10RB and therefore there could be a steric hindrance effect. To solve this, we designed different circular permutants of Foldikine10 and we found as indicated above one (Foldikine10_cut116 (SEQ ID NO: 5)) that was expressed at WT levels and had around three times worst EC-50 (See Figure 4). In this circular permutant, the Nt and Ct point not to the membrane and are far away from the receptors, thus we expect no steric hindrance when fused to CSF1. To see if this is the case, we fused Foldikine10_cut116 to CSF1 in both orientations (N-terminus and C-terminus: Foldikine10_cut116-CSF1-HiBit (SEQ ID NO: 56); CSF1-Foldikine10_cut116-HiBit (SEQ ID NO: 57)). The fusion of the circular permutant compared to the CSF1-Foldikine10 scaffold resulted in similar CSF1 EC-50 values in both orientations (see Figures 15-16). We observed that the EC-50 for IL10 of the Foldikine circular permutation alone (Foldikine_cut116) or in combination with CSF1 has similar EC-50 values, in contrast with the behavior of Foldikine10 that loses activity when fused to CSF1, supporting the hypothesis of the steric hindrance effect mentioned above (see Table 9).

**Table 9.** EC-50 values and the error associated for both CSF1 and IL10 activity.

| Circular permutant | CSF-1 | | IL-10 | |
|---|---|---|---|---|
| | EC-50 | error EC-50 | EC-50 | error EC-50 |
| CSF1-foldikine10_cut116 | 4.14E-09 | 3.38E-10 | 3.06E-10 | 1.73E-11 |
| foldikine10_cut116-CSF1 | 2.69E-09 | 2.13E-10 | 4.95E-10 | 2.01E-11 |
| CSF1-Foldikine10 | 3.62E-09 | 2.60E-10 | 1.67E-10 | 3.29E-11 |
| Foldikine10 cut_116 | | | 2.41E-10 | 2.08E-11 |

***Impact of linker length in protein functionality***

We tested Orientation B (CSF-linker-IL10) to determine whether shorter or longer linkers might interfere with protein functionality. While CSF1-Foldikine10 showed IL-10 signaling with linker lengths ranging from 15 to 35 amino acids (aa) (CSF1-25AA-Foldikine10-HiBit (SEQ ID NO: 58); CSF1-30AA-Foldikine10-HiBit (SEQ ID NO: 55); CSF1-35AA-Foldikine10-HiBit (SEQ ID NO: 59); CSF1-15AA-Foldikine10-HiBit (SEQ ID NO: 60)), CSF1-IL-10 with a short linker (i.e., 15 aa: CSF1-15AA-IL10-HiBit (SEQ ID NO: 61)) was not active. CSF1-IL-10 with linker lengths ranging from 25 to 35 amino acids showed IL-10 signaling (CSF1-25AA-IL10-HiBit ((SEQ ID NO: 62), CSF1-30AA-IL10-HiBit ((SEQ ID NO: 63), CSF1-35AA-IL10-HiBit ((SEQ ID NO: 64)). In terms of CSF1 activity, the protein expression for two mutants (CSF1-15AA-IL10-HiBit (SEQ ID NO: 61) and CSF1-25AA-IL10-HiBit (SEQ ID NO: 62)) was below the dynamic range of the CSF1R HEK reporter cells. The fusion CSF1-Foldikine10 supports different linker lengths, whereas IL-10 either loses activity or expression with short linkers. See Figures 17-18 and Tables 10 and 11.

**Table 10.** EC-50 values and the error associated for IL10 activity fused with linkers of different length.

| | CSF1-15AA-IL10 | CSF1-25AA-IL10 | CSF1-30AA-IL10 | CSF1-35AA-IL10 | CSF1-15AA-Foldikine10 | CSF1-25AA-Foldikine10 | CSF1-30AA-Foldikine10 | CSF1-35AA-Foldikine10 |
|---|---|---|---|---|---|---|---|---|
| EC-50 | | 4.10E-10 | 2.35E-10 | 1.9E-10 | 2.60E-10 | 3.61E-10 | 3.32E-10 | 3.18E-10 |
| Error | | 2.11E-11 | 1.57E-11 | 1.23E-11 | 6.61E-12 | 2.81E-12 | 6.74E-12 | 2.35E-12 |

The Hill slope was fixed to -1.2 and the baseline to 0.40. *IL10 concentration of the active dimeric form estimated by Hibit is double than for Foldikine10, thus we divided the EC50 by two.

**Table 11.** EC-50 values and the error associated for CSF1 activity fused with linkers of different length.

| | CSF1-30AA-IL10 | CSF1-35AA-IL10 | CSF1-15AA-Foldikine10 | CSF1-25AA-Foldikine10 | CSF1-30AA-Foldikine10 | CSF1-35AA-Foldikine10 |
|---|---|---|---|---|---|---|
| EC-50 | 5.50E-10 | 6.50E-10 | 1.19E-09 | 1.64E-09 | 1.97E-09 | 1.83E-09 |
| Error | 2.79E-10 | 2.50E-10 | 3.21E-10 | 4.84E-10 | 4.76E-10 | 4.39E-10 |

The Hill slope was fixed to -1.06 and the baseline to 1.2

## Example 2: Methods

### Generation of circular permutant molecules using FoldX-ModelX software

Circular permutants are generated in 2 steps: first by linking natural Nt-Ct a cyclic structural model with no beginning and no end is generated, second by disconnecting a peptide bond in a flexible region of the model. Linkers between Nt-Ct are engineered using *Bridging* from ModelX. *AlaScan* from FoldX, helps in the selection of cutting residues predicting those that are less important for the stability of the protein.

### Generation of single-chain (SC) molecules using ModelX software

*SC-IL10 variants* were generated by rewiring the X-ray IL-10 dimeric structure (PDBs: 1y6k, 2ilk). The new connectivities were designed using the ModelX tool suite. The ModelX *Bridging* command (cross-linking mode) was used, which connects a pair of residues selected as anchors with all geometrically compatible peptidic fragments from a custom made protein fragment library (PepXDB). PepXDB 120k structures were extracted from PDB and digested into peptides of different lengths (3-45 aa) and included geometrical descriptors needed for the *Bridging* algorithm. The *Bridging* command allows the user to select different peptide lengths; the output is an ensemble of bridged models with different conformations, sequences, and lengths, whereby linkers/connections with forbidden phi and psi dihedrals in the Ramachandran plot are discarded. Once the sewing patterns are created, an extensive linker screening is performed by running them through the *Bridging* algorithm with exhaustive combinations of anchoring points in an overlapping sliding window around the flanks of the regions to be joined. Every window queried for different peptide lengths (6-20 aa). The linkers on the bridged models contain the same side-chain rotamers that they had on the PDB structure from which they were digested; thus, adaptation to the new SC-IL10 context is done using the RepairPDB command of FoldX (Montero-Blay et al. 2023, Molecular Systems Biology, 19:e11037). RepairPDB identifies residues that have bad torsion angles, VanderWaals' clashes, or total energy, and mutates them and their neighbours to themselves, exploring different rotamer combinations to find new energy minima. The resulting models are ranked by global energy (FoldX *Stability* command). When peptides are longer than numeric positions between the anchors, *Bridging* renumbers surplus residues with res codes that are not recognized by FoldX in further modelling steps. For this reason, SC-IL10 design required a numerical rearrangement of the monomeric protein residues in the dimer, allowing numeric 18-aa-long gaps between the regions to be connected. This is very important for the extreme case in which anchoring residues flank the numeric gap. To cover that situation, renumbering was done to create gaps of 18 numeric positions around the regions to be connected, which was enough to accept length fragments ranging from 6- to 20-residues without using the residue codes. The difference between 18 and 20 is that anchoring points are required for the algorithm to position the bridges, but they will be substituted with the terminal residues of the peptides found. *Bridging* also replaces all fringe residues between the anchors with the ones from the peptide. For clarity, the templates generated before using *Bridging* are termed "sewing patterns". As the numeric gap is not a spatial gap, *Bridging* can also accept smaller peptide fragments, creating a discontinuous numbering.

### Linker generation to link Foldikine10 variants with CSF1

To generate the linker between the Foldikine10 variants and the CSF1, we first looked at the 3D structure of the complexes between IL-10 and IL-10 RA and RB (PDB 6x93; 3.5 Å) and of CSF1 with its receptor (PDB 4wrm; 6.5 Å) placing the receptors in the orientation we would expect them to have on the membrane and determine the distance between the natural Nt or CT of IL10 with the corresponding Ct or NT of CSF1. Using this distance as a proxy we estimated that we needed a random linker between CSF1 Cys146 and IL10 Cys12 aa composed of repeated GGSGG (SEQ ID NO: 27) units of around 30 aminoacids.

### Generation of affinity mutants in IL-10 and CSF1 using FoldX software

A) IL-10. To generate IL-10 versions with a decreased affinity towards R1, we used the hIL-10 molecules crystallized in complex with IL-10RA with the best crystal resolution (PDB 1y6k; 2.5Å). We then performed an *in silico* mutagenesis scanning of the complex interface residues using the BuildModel FoldX command, which individually mutates all IL-10 residues to the other 19 natural amino acid (Schymkowitz et al, 2005), and the AnalyseComplex FoldX command to estimate the change in the interaction energy of each mutation. The selection of mutants was based on complex interaction energy (ΔΔG) and stability predicted by FoldX, and visual inspection of the complex interface. Of this we selected R24A, Q34A, M39A, D44N, D44A, L46A, E50A, E50Y, E151R, D144Q.

B) CSF1. To generate mutants of CSF1 with increased affinity towards its receptor we used the CSF1 crystallized with CSF1 (PDB 4wrl; 2.8 Å). We then performed an *in-silico* mutagenesis scanning of the complex interface residues using the BuildModel FoldX command, which individually mutates all CSF1 residues to the other 19 natural amino acid (Schymkowitz et al, 2005, PNAS, 102(29), 10147-10152), and the AnalyseComplex FoldX command to estimate the change in the interaction energy of each mutation. We select a set of conserved and non-conserved residues based on complex interaction energy (ΔΔG) and stability predicted by FoldX, and visual inspection of the complex interface. Of this we selected H9F, Q17R, S18Q, V78W, Q79L, Q81F, Q81Y, E82K for a first round of mutants and V78F, S18F-R21Q, Q17V/L, S18F, V78F/L/Y, Q79T/K, K93E, T93E, T34L and M10L for a second round.

### Generation of stability mutants in CSF1 using FoldX software

We used the CSF1 crystallized with CSF1 (PDB 4wrl; 2.8 Å) to generate mutants of CSF1 with increased stability of CSF1 towards CSF1R in the bound state. We then performed an in-silico mutagenesis scanning of the complex interface residues using the BuildModel FoldX command, which individually mutates all CSF1 residues to the other 19 natural amino acid (Schymkowitz et al., 2005, PNAS, 102(29), 10147-10152). Based on complex stability predicted by FoldX, and visual inspection we select A76I, V117I, V117L, V120I, T124I.

### Protein quantification

Since we generated different point mutations to improve or decrease affinity to the receptors as well as we have broken loops in some of the constructs or made new linkers there is a danger that the resulting molecules will not be recognized by the antibody used in commercial ELISA kits. To avoid this issue, we have used the HIBIT approach to quantify all of our constructs (https://www.promega.es/en/resources/technologies/hibit-protein-tagging-system/). Essentially this involves fusing a peptide tag spaced by a random linker to the Nt or CT of aThe Nano-Glo® HiBiT Extracellular Detection System is a method that can detect HiBiT-tagged proteins secreted into the extracellular medium. The detection is done by adding a nonlytic detection reagent containing a substrate and Large BiT (LgBiT). HiBiT binds tightly to LgBiT, promoting complex formation and generating a bright, luminescent enzyme. The amount of luminescence generated is proportional to the amount of HiBiT-tagged protein present, and the system can detect protein amounts over seven orders of magnitude. The detection process is simple and requires only an add-mix-read assay protocol.). By having a peptide reference of know concentrations it is extremely easy to quantify the expression of any protein.

### Plasmid generation

All plasmids generated in this work were assembled following the Gibson method (Gibson DG et al. Nat Methods. 2009 May;6(5):343-5). When required, Integrated DNA Technologies (IDT) Corporation performed gene synthesis (gBlock double-stranded fragments) and oligonucleotides synthesis. Gene amplification was carried out with Phusion DNA polymerase (Thermo Fisher Scientific) and transformed in Escherichia coli DH5-alpha competent cells (NEB).

For mammalian protein expression in ExpiCHO cells, the vector used was pcDNA3.1 (V790-20, Invitrogen). All the plasmids were verified by Sanger sequencing (Eurofins Genomics).

### Expression in CHO cells

ExpiCHO protein expression kit cells were purchased from Thermofisher (A29133). For a low-scale production (2.5 mL), 24 deep well plates were used (AXYPDW10ML24CS, Merck) and covered by gas-permeable film (Thermo-Fisher). The day -1 of production, ExpiCHO-cells were split to a final density of $3 \times 10^6$ viable cells/mL. Grown at 37C, 8% $CO_2$ and shaking at 110rpm. The Day 0 of production, ExpiCHO cells were split to a final density of $3 \times 10^6$ viable cells/mL per sample aliquot 2.5mL of the cell suspension into one well of the plate. Per sample, 2ug of DNA to 200ul of cold OptiPRO SFM & 9.0 μL ExpiFectamine CHO Reagent was added. Then, 200 μL of the complexation mixture was added to each relevant well. All the plate was covered with a gas permeable seal and placed at 37°C, 8% $CO_2$ and 225rpm. The day 1 after 18-22 hours post-transfection, ExpiFectamine CHO Enhancer and ExpiCHO Feed was added to each well. For an entire plate of 24 wells, 400 μL ExpiFectamine CHO Enhancer and 16 mL ExpiCHO Feed in a conical tube were mix. From

this mixture, 600 μL was added to each well of the plate. Place at 37C, 8% CO2 and 225rpm. After 4 days the samples were harvested. Plates were centrifuged 5 minutes at 4600 rpm at room temperature. Supernatants were aliquoted, snap frozen liquid nitrogen and stored at -80°C.

**Cell culture**

The HEK-Blue™ cell lines carrying a SEAP reporter construct were purchased from InvivoGen (InvivoGen, San Diego, CA, USA). In this work we used Human IL-10 Reporter Cells (hkb-il10). HEK293 cells were directly purchased to InvivoGen. Both HEK-Blue™ cell lines and HEK293 cells were grown in DMEM (Lonza, BE12-604F) supplemented with 10% FBS, 2 mM L-glutamine and specified selection antibiotics by the manufacturer in each of the reporter cells. Cells were passed when 70% confluence was reached, following the manufacturer's recommendation.

We culture THP-1 (ATCC, lot: 70047949) cells in RPMI-1640 with 10% Fetal Bovine serum, 2-mercaptoethanol to a final concentration of 0.05 mM and Pen Strep (Gibco/15140122) at 37°C in a CO2 incubator. We maintain cell concentration between 0.2-1e6 cells/ml.

**Colorimetric analysis in HEK-Blue™ Cells for Foldikine-10 activity**

After supernatant quantification, samples were adjusted to fit into the dynamic range of the HEK reporter cells using DMEM media for performing the different dilutions. Each of the top concentration was set for each reporter cell to match saturation, and 8 serial dilutions were performed (0.5X each) for each sample. Then, HEK reporter cells were prepared according to manufacturer instructions. Briefly, 180 μL of cells at 280,000 cells/mL per well were seeded in a 96-well plate (Nunc Microwell, ThermoFisher Scientific, #167008). From this, 20 μL of each supernatant with adjusted concentration was added, and cells were kept between 20-24 h at 37°C and 5% CO2 (induced HEK-BlueTM). After, 180 μL of QUANTI-Blue Solution (Alkaline phosphatase detection medium, #repqbs, InvivoGen) was mixed with 20-μL induced HEK-BlueTM cells in a new 96-well plate. Cells were then incubated 60 min at 37°C, and absorbance (630 nm) was measured in the spectrophotometer Tecan i-control, 2.0.10.0.

**HEK293 CSF1 Reporter cells**

Colorimetric analysis in HEK-Blue™ Cells for CSF1 activity CSF1R/SER Reporter kit (MAPK/ERK Signaling Pathway, #79379) was purchased from BPS Bioscience (BPS Bioscience Inc, San Diego, CA, USA). The day before the DNA transfection, HEK293T cells (3x104 cells/well) were seeded in a 96 multi-well plate (Nunc Microwell, ThermoFisher Scientific, #167008) in 100 μl of DMEM (Lonza, BE12-604F) supplemented with 10% FBS, 2 mM L-glutamine and 1% penicillin/streptavidin and incubated for 20-24h at 37°C and 5% CO2. The day after, we prepared 100 μl of SER luciferase reporter vector (inducible luciferase vector), 100 μl of CSF1R expression vector (plasmid C) and 1500 μl of Opti-MEM (GIBCO, #31985-070). In parallel 30 μl of Lipofectamine 2000 (Invitrogen, #11668019) were diluted in 1500 μl of Opti-MEM (GIBCO, #31985-070). After 5 minutes incubation, the diluted DNA with diluted Lipofectamine 2000 were combined, mixed gently and incubated for 25 minutes at room temperature to form the complexes. After that time 30 μl of the complexes were added to each well containing the cells. Cells were incubated at 37°C in a 5% CO2 incubator. After ~5 to 6 hours of transfection, medium was changed to growth medium with 0.5% serum. Incubate cells at 37°C in a 5% CO2 incubator overnight. The day after, the SRE reporter was induced by activating the CSFR1R signaling cascade with our CSF1-IL10 fusion variants. After 6-hour treatment, remove the media and add 50 μl of diluted Passive Lysis Buffer 5X (Promega, #E1941). 100x Firefly Luciferase Reagent Substrate (Component B) was diluted into Firefly Luciferase Reagent Buffer (Component A), 20 μl of this mix was added on top of 20 μl of your lysate placed in a white 96 multi-well plate (PerkinElmer, #6005290) and the plate was incubated at room temperature for 15 minutes. Then firefly luminescence was measured using a luminometer. Afterwards, 20 μl of diluted 100x Renilla Luciferase Reagent Substrate (Component D) into Renilla Luciferase Reagent Buffer (Component C) was added to the well and immediately after luminescence was measured using a luminometer. To obtain the normalized luciferase activity for the SRE reporter, we calculate the ratio of firefly luminescence to renilla luminescence and inferred a dose-response linear fit (Hill slope), see below.

**Inferring EC-50 data**

For the inference of an EC-50, we measured a dose-response analysis by following up absorbance (HEKBlue10) or fluorescence (HEK293-CSF1 reporter).

The changes in absorbance at 630 nm due to different IL-10 tested concentrations were fitted to a saturation binding model using the following equation:

$$Y = \text{Baseline} + (\text{B Max} - \text{Baseline})/(1+(X/EC50)^{\wedge} \text{-Hill-slope}).$$

In this equation, EC-50 is the apparent dissociation constant (as the number of active receptors per cell is unknown), h is the Hill-slope, and B Max is the saturation signal. This equation assumes specific binding only; all non-specific signals were subtracted. For EC-50 calculated in Table 3 and 6 the equation used was log(agonist) vs. response -variable slope (Y=Bottom + (Top-Bottom)/(1+10^((LogEC50-X)*HillSlope)) from Prism 9 software.

For the CSF1 analysis we measured changes in the ratio of firefly/renilla luminescence.

The different experiments were analysed independently and fitted using GraphPad Prism 9 software. For IL-10 and Foldikin10 variants in those experiments were the basal or final lines of some mutants were not defined we used the average values from those mutants with lower EC-50 (final value at saturation) or high EC-50 (initial value with no ligand). In the case of CSF1 since we cannot be sure that the transformation efficiency with the CSFR1 kit was the same in all cases, we fix the initial value as indicated above and we fix the Hill slope to the average of the mutants with lower EC-50. In each Figure we indicated how the fitting was done.

### *Testing the activity of the CSF1-Foldikine in THP-1 cells*

Thp1 cells were prepared at a concentration of 1e6/mL live cells. Then, 100 $\mu$l of cells/well were plated in a 96-well U-shaped plate and incubated for 15 minutes. In parallel, supernatants at the targeted dilution (60nM) were prepared in a pre-warmed 96-well plate with RPMI medium. Next, 20 $\mu$l of the dilutions were added to 100 $\mu$l of cells and incubated for 20 minutes. Afterwards, the cells were immediately fixed to maintain the phosphorylation state by adding an equal volume of pre-warmed BD Cytofix Buffer to the cell suspension, resulting in a total volume of 240 $\mu$l. The cells were incubated at 37°C for 10 minutes and then pelleted by centrifugation. Subsequently, the cells were permeabilized by adding 100 $\mu$l of BD™ Phosflow Perm Buffer III (for $1\times106$ cells) and incubating for 30 minutes on ice (protected from light). Note: Longer incubation times in this permeabilization buffer may decrease the signal intensity of surface marker staining. Then, the cells were washed twice with 200 $\mu$l of wash buffer (1X PBS + 1% FBS + 0.09% Sodium Azide) and centrifuged to remove the supernatant. After that, 5 $\mu$l of the fluorochrome-conjugated antibody Alexa Fluor 488 pSTAT3 Y407 was added to each well of the U-bottom 96-well plate. The plate was incubated at room temperature for 60 minutes in the dark. Finally, the cells were washed and resuspended in PBS for flow cytometry analysis.

### Example 3: *in vitro* functional evaluation of the dual CSF1-IL10 cytokine

For the experiments of examples 3 and 4, FLDK1 (SEQ ID NO: 72, i.e. SEQ ID NO:16 or 55 with tag HisHiBit), and/or CSF1-IL-10 (SEQ ID NO:74) was used as exemplary dual CSF1-IL10 cytokine.

PBMCs (150,000 cells/well) from healthy donors were stimulated with 10 ng/ml of LPS (Sigma) in the presence or absence of different concentrations of rIL-10 or FLDK-1 (0.00001 to 1 nM) or of CSF1-IL-10 (Fig. 20 A). The TNF$\alpha$ levels measured in the presence of rIL-10 or FLDK-1, show that FLDK-1 inhibits monocytes activation similarly to IL-10 (dotted line indicates the levels of LPS alone) (Fig. 20 B). CSF1-IL-10 was also found to inhibit monocytes activation (Fig. 20 C).

Similar results obtained with IL-6 and when using isolated monocytes (CD14+ cells) (data not shown).

Additionally, PBMCs were isolated from human buffy coats and CD14+ cells were purified by positive microbead selection (Miltenyi). Monocytes (0.5 million/ml) were cultured for 7 days in the presence or absence of: rIL-10, rCSF1, FLDK1 (10 ng/ml per treatment) or untreated condition. Cytokines were added every 2 days. At day 7 alive cells were counted and characterized by FACS (CD163, CD206, MHCII, CD14, CD86) (Fig. 21 A). The results show that FLDK1, similarly to CSF1, induces the differentiation of monocyte-derived macrophages, as identified by the CD163 expression (Fig. 21 B, C).

Further characterization was performed to determine the phenotype of the induced monocyte-derived macrophages.

PBMCs were isolated from human buffy coats and CD14+ cells were purified by positive selection (Miltenyi). Monocytes (0.5 million/ml) were cultured for 7 days in the presence or absence of: rCSF1 or FLDK1 (10 ng/ml). Cytokines were added every 2 days. At day 7, cells were harvested and stimulated with LPS, with or without IFN$\gamma$ (Fig. 22 A). TNF-$\alpha$ levels in the supernatants were determined by ELISA, 17h after stimulation. CSF-1-differentiated macrophages produce TNF-$\alpha$, which is further enhanced by the addition of IFN$\gamma$. In contrast, FLDK1-differentiated macrophages do not produce TNF-$\alpha$, even when stimulated with LPS+IFN$\gamma$. These results show that FLDK 1-induced macrophages have a regulatory phenotype (Fig. 22 B).

Additionally, monocytes were isolated from human buffy coat samples by selecting for positive CD14 cells (Miltenyi). Monocytes (0.5 million/ml) were cultured for 7 days in the presence or absence of rCSF1 or FLDK1 (10 ng/ml per treatment). At day 7, cells were detached from plates and counted, same number of macrophages (40,000 cells) were replated and stimulated with LPS (Sigma) (10 ng/ml) for 4 hours. CD3+ cells from a different donor were purified, counted (120,000 cells) and added to CSF1 or FLDK1-differentiated macrophages. Cytostim (Miltenyi) (2 µl/million of cells) was also added to the co-cultures to enhance the interaction between both cell types. Cytokines in the supernatants were analyzed by ELISA at 48h and proliferation of T cells was analyzed after 5 days in co-culture (Fig. 23 A). FLDK1-differentiated macrophages, inhibit the proliferation of T cells and the production of TNF-α and IFNγ in these cocultures, demonstrating their regulatory phenotype. (Fig. 23 B).

PBMC (150,000 cells/well) from healthy donors were plated on in 96-well plates and stimulated next day with rIL10 or FLDK1 (10 nM per treatment, 8 dilutions by 1/3) for 20 min at 37°C. Cells were stained with CD14 Alexa647 (Biolegend) and CD3 APC/Cy7 (Biolegend), fixed with 4% PFA and permeabilized for intracellular staining with 80% MetOH. Cells were stained with AlexaFluor488 pSTAT3 (pY705) (Becton Dickinson) for 1 hour (Fig. 24 A). Samples were analyzed with LSRII cytometer and MFI values obtained from STAT3 activation were plotted using GraphPad Prism software. One representative result with two technical replicates is shown as mean with error bar depicting the SEM (Fig. 24 B-C). Similar results were obtained in 10 different donors. Each biological replicate was normalized by assigning the highest IL10 MFI value of the top concentration as 100% and the lowest MFI value of an untreated control as 0%. The MFI from the samples treated with FLDK1 was normalized accordingly. These results indicate that both monocytes and T cells respond in similar way to IL-10. In contrast, the dual CSF1-IL10 cytokine FLDK-1 preferentially activates monocytes when compared to T cells (Fig. 24 B-C). These results show that FLDK1 shows selectivity towards myeloid cells compared to lymphoid cells, in contrast with IL-10.

FLDK-1 and IL-10, as well as CSF1-IL-10 and IL-10 respective potencies at activating CD8 T and B cells were further characterised.

T cells were isolated from human PBMCs using positive microbeads selection (Miltenyi). The lymphocytes (1.5 millions/ml) were stimulated with anti-CD3 (ThermoFisher) (1 µg/ml) and anti-CD28 (ThermoFisher) (3 µg/ml) for 72 hours. Then, cells were splited in two and treated with IL10, or FLDK1 or CSF1-IL10 (10 nM per treatment) with IL-2 (5 ng/ml) for 48 hours. Cell supernatants were obtained and Granzyme B (Gzmb) and IFNγ were detected by ELISA (Fig. 25 A). Pooled normalized data of 4 independent experiment are shown in the graph for Gzmb. t-test IL-10 vs FLDK1 p-value < 0.05. The results show that FLDK-1 (Fig. 25 B) or CSF1-IL-10 (Fig. 25 C) is less potent than IL-10 at activating CD8 T cells.

B cells were isolated from human PBMCs using positive microbeads selection (Miltenyi). The isolated lymphocytes (50,000 cells/well) were stimulated with CpG (InvivoGene) (1 µg/ml) and IL10, or FLDK1 or CSF1-IL10 (10 nM per treatment) at days 0 and 2. After 7 days cell supernatants were obtained for the determination of IgG levels by ELISA (Fig. 26 A). Similar results were obtained when stimulating with CD40L. Pooled data of 4 independent experiment is shown in the graph (Fig. 26 B). t-test IL-10 vs FLDK1 p-value < 0.05. The results show that FLDK-1 Fig. 26 B) or CSF1-IL-10 (Fig. 26 C) is less potent than IL-10 at activating B cells.

**Example 4: *in vivo*, the dual CSF1-IL10 cytokine alleviates clinical symptoms in a mouse model of colitis and other inflammatory diseases**

Evaluation of the therapeutic efficacy of FLDK1 (in three doses) was performed in an experimental model of chronic IBD induced by TNBS (Trinitrobenzene sulfonic acid (TNBS)-induced colitis mouse model), with comparison with a molecule of reference (recombinant IL-10).

TNBS was intrarectally infused in male Balb/c mice at four increasing doses (0.7 mg/mouse at day 0; 0.8 mg/mouse at day 8; 1.0 mg/mouse at day 15; and 1.2 mg/mouse at day 22). Measurements of body weight were performed daily.

The experimental Groups included 10 mice per group and were as follows:

1. 50% Ethanol by intrarectal route
2. TNBS/50% ethanol + treatment with vehicle by intrarectal route
3. TNBS/50% ethanol + treatment with FLDK1 (dose 1 µg per mice) by intrarectal route
4. TNBS/50% ethanol + treatment with FLDK1 (dose 3 µg per mice) by intrarectal route
5. TNBS/50% ethanol + treatment with FLDK1(dose 10 µg per mice) by intrarectal route
6. TNBS/50% ethanol + treatment with rIL10 (dose 1 µg per mice) by intrarectal route
7. TNBS/50% ethanol + treatment with rIL10 (dose 3 µg per mice) by intrarectal route
8. TNBS/50% ethanol + treatment with rIL10 (dose 10 µg per mice by intrarectal route

FLDK1 and rIL10 were administered intraperitoneally, at every dose, 3 times per week (every other day, starting the day of each TNBS infusion).

The results show that mice treated with FLDK1 had less body weight loss compared to both IL10-treated or vehicle-treated mice, after 30 days treatment, in a dose-response manner (Fig. 27 A-C).

**Example 5: the selectivity of the dual CSF1-IL10 cytokine for monocytes versus T cells is mediated by the CSF1 part of the cytokine**

A structure-activity evaluation of FLDK1 was conducted as follows.

PBMCs (150,000 cells/well) from healthy donors were plated on 96-well plates and stimulated next day with FLDK1, IL-10 or FLDK1-E82A (SEQ ID NO: 73) (10nM per treatment) (a mutated version of FLDK1 in which CSF1 cannot bind CSF1-R due to the mutation E82A) for 20 min at 37°C (Fig. 28 A). Cells were stained with CD14 Alexa647 (Biolegend) and CD3 APC/Cy7 (Biolegend), fixed with 4% PFA and permeabilized for intracellular staining with 80% MetOH. Cells were stained with AlexaFluor488 pSTAT3 (pY705) (Becton Dickinson) for 1 hour. Samples were analyzed with LSRII cytometer and MFI values obtained from STAT3 activation were plotted using GraphPad Prism software. One representative result with two technical replicates is shown as mean with error bar depicting the SEM. Each condition was normalized by assigning the highest **IL10** MFI value of the top concentration as 100% and the lowest MFI value of an untreated control as 0%. The MFI from the rest of the samples treated was normalized accordingly.

The results show that the selective activation of monocytes by FLDK1 is mediated by the CSF-1 part of the cytokine as the selective activation of monocytes versus T cells is greatly reduced with mutant FLDK1-E82A (Fig. 28 B).

PBMC (150,000 cells/well) from healthy donors were also plated on 96-well plates and stimulated next day with rIL10, FLDK1 (10nM per treatment), or saturating concentrations of CSF1 (100 nM) (for 2 min) followed by FLDK1 (10 nM) for 20 min at 37°C (Fig. 29 A). Cells were stained with CD14 Alexa647 (Biolegend) and CD3 APC/Cy7 (Biolegend), fixed with 4% PFA and permeabilized for intracellular staining with 80% MetOH. Cells were stained with AlexaFluor488 pSTAT3 (pY705) (Becton Dickinson) for 1 hour. Samples were analyzed with LSRII cytometer and MFI values obtained from STAT3 activation were plotted using GraphPad Prism software. One representative result with two technical replicates is shown as mean with error bar depicting the SEM. Each condition was normalized by assigning the highest IL10 MFI value of the top concentration as 100% and the lowest MFI value of an untreated control as 0%. The MFI from the rest of the samples treated was normalized accordingly.

The results show that the selective activation of monocytes by FLDK1 is mediated by the CSF-1 part of the cytokine as saturating concentrations of CSF-1 prevents the selective activation of monocytes versus T cells by FLDK1 (Fig. 29 B).

**SEQUENCES**

NtCt linkers are underlined. Intermolecular linkers are shown underlined and in italic. Mutations are in bold. Tags like HiBit or TevHisHibit for protein quantification are shown in bold italics as they are the Gly linkers between them. Signal peptides used to secrete the proteins which are cleaved upon secretion are not showed. We used for proteins starting by IL10 and Foldikine10 the signal sequence: MHSSALLCCLVLLTGVRA (SEQ ID NO: 69). In the case of proteins starting by CSF1 we used MTAPGAAGRCPPTTWLGSLLLLVCLLASRSIT (SEQ ID NO:70).

>SEQ ID NO: 1 foldikine10:

**M**TQSENSCTHFPGNLPNMLRDLRDAFSRVKTFFQMKDQLDNLLLKESLLEDFKGYLGCQA
LSEMIQFYLEEVMPQAENQ**E**PDIKAHVNSLGENLKTLRLRLRRCHRFLPCENKSKAVEQV
KNAFNKLQEKGIYKAMSEFDIFINYIEAYMTM**N**<u>NGGLDY</u>LPNMLRDLRDAFSRVKTFFQM
KDQLDNLLLKESLLEDFKGYLGCQALSEMIQFYLEEVMPQAENQDPDIKAHVNSLGENLK
TLRLRLRRNHRFLPCENKSKAVEQVKNAFNKLQEKGIYKAMSEFDIFINYIEAYMTMKIR
N

>SEQ ID NO: 20 foldikine10-HiBit:

**M**TQSENSCTHFPGNLPNMLRDLRDAFSRVKTFFQMKDQLDNLLLKESLLEDFKGYLGCQA
LSEMIQFYLEEVMPQAENQ**E**PDIKAHVNSLGENLKTLRLRLRRCHRFLPCENKSKAVEQV
KNAFNKLQEKGIYKAMSEFDIFINYIEAYMTM**N**<u>NGGLDY</u>LPNMLRDLRDAFSRVKTFFQM
KDQLDNLLLKESLLEDFKGYLGCQALSEMIQFYLEEVMPQAENQDPDIKAHVNSLGENLK
TLRLRLRRNHRFLPCENKSKAVEQVKNAFNKLQEKGIYKAMSEFDIFINYIEAYMTMKIR
N***GGVSGWRLFKKIS***

>SEQ ID NO: 2 ORK10-002

**M**TQSENSCTHFPGNLPNMLRDLRDAFSRVKTFFQMKDQLDNLLLKESLLEDFKGYLGCQA
LSEMIQFYLEEVMPQAENQDPDIKAHVNSLGENLKTLRLRLRRCHRFLPCENKSKAVEQV
KNAFNKLQEKGIYKAMSEFDIFINYIEAYMTM**N**FGGLDYLPNMLRDLRDAFSRVKTFFQM
KDQLDNLLLKESLLEDFKGYLGCQALSEMIQFYLEEVMPQAENQDPDIKAHVNSLGENLK
TLRLRLRRCHRFLPCENKSKAVEQVKNAFNKLQEKGIYKAMSEFDIFINYIEAYMTM

>SEQ ID NO: 3 ORK10-003

**M**TQSENSCTHFPGNLPNMLRDLRDAFSRVKTFFQMKDQLDNLLLKESLLEDFKGYLGCQA
LSEMIQFYLEEVMPQAENQDPDIKAHVNSLGENLKTLRLRLRRCHRFLPCENKSKAVEQV
KNAFNKLQEKGIYKAMSEFDIFINYIEAY**L**YKTIT**PEFA**NMLRDLRDAFSRVKTFFQMKD
QLDNLLLKESLLEDFKGYLGCQALSEMIQFYLEEVMPQAENQDPDIKAHVNSLGENLKTL
RLRLRRCHRFLPCENKSKAVEQVKNAFNKLQEKGIYKAMSEFDIFINYIEAYMTM

>SEQ ID NO: 49 ORK10-003-HiBit

**M**TQSENSCTHFPGNLPNMLRDLRDAFSRVKTFFQMKDQLDNLLLKESLLEDFKGYLGCQA
LSEMIQFYLEEVMPQAENQDPDIKAHVNSLGENLKTLRLRLRRCHRFLPCENKSKAVEQV
KNAFNKLQEKGIYKAMSEFDIFINYIEAY**L**YKTIT**PEFA**NMLRDLRDAFSRVKTFFQMKD
QLDNLLLKESLLEDFKGYLGCQALSEMIQFYLEEVMPQAENQDPDIKAHVNSLGENLKTL
RLRLRRCHRFLPCENKSKAVEQVKNAFNKLQEKGIYKAMSEFDIFINYIEAYMTM***GGVSG***
***WRLFKKIS***

>SEQ ID NO: 4 ORK10-005

**M**TQSENSCTHFPGNLPNMLRDLRDAFSRVKTFFQMKDQLDNLLLKESLLEDFKGYLGCQA
LSEMIQFYLEEVMPQAENQDPDIKAHVNSLGENLKTLRLRLRRCHRFLPCENKSKAVEQV
KNAFNKLQEKGIYKAMSEFDIFINYIEA**K**DKDIRDGD**ELA**NMLRDLRDAFSRVKTFFQMK
DQLDNLLLKESLLEDFKGYLGCQALSEMIQFYLEEVMPQAENQDPDIKAHVNSLGENLKT
LRLRLRRCHRFLPCENKSKAVEQVKNAFNKLQEKGIYKAMSEFDIFINYIEAYMTM

>SEQ ID NO: 5 Foldikine_cut116

NKSKAVEQVKNAFNKLQEKGIYKAMSEFDIFINYIEAYMTM**N**NGGLDYLPNMLRDLRDAF
SRVKTFFQMKDQLDNLLLKESLLEDFKGYLGCQALSEMIQFYLEEVMPQAENQDPDIKAH
VNSLGENLKTLRLRLRRNHRFLPCENKSKAVEQVKNAFNKLQEKGIYKAMSEFDIFINYI
EAYMTM**N**NGGLDYLPNMLRDLRDAFSRVKTFFQMKDQLDNLLLKESLLEDFKGYLGCQAL
SEMIQFYLEEVMPQAENQDPDIKAHVNSLGENLKTLRLRLRRNHRFLPCE

>SEQ ID NO: 6 Foldikine cut133

EKGIYKAMSEFDIFINYIEAYMTM**N**NGGLDYLPNMLRDLRDAFSRVKTFFQMKDQLDNLL
LKESLLEDFKGYLGCQALSEMIQFYLEEVMPQAENQDPDIKAHVNSLGENLKTLRLRLRR
NHRFLPCENKSKAVEQVKNAFNKLQEKGIYKAMSEFDIFINYIEAYMTM**N**NGGLDYLPNM
LRDLRDAFSRVKTFFQMKDQLDNLLLKESLLEDFKGYLGCQALSEMIQFYLEEVMPQAEN
QDPDIKAHVNSLGENLKTLRLRLRRNHRFLPCENKSKAVEQVKNAFNKLQ

>SEQ ID NO: 7 Foldikine_cut209

GYLGCQALSEMIQFYLEEVMPQAENQDPDIKAHVNSLGENLKTLRLRLRRNHRFLPCENK
SKAVEQVKNAFNKLQEKGIYKAMSEFDIFINYIEAYMTM**N**NGGLDYLPNMLRDLRDAFSR
VKTFFQMKDQLDNLLLKESLLEDFKGYLGCQALSEMIQFYLEEVMPQAENQDPDIKAHVN
SLGENLKTLRLRLRRNHRFLPCENKSKAVEQVKNAFNKLQEKGIYKAMSEFDIFINYIEA
YMTM**N**NGGLDYLPNMLRDLRDAFSRVKTFFQMKDQLDNLLLKESLLEDFK

>SEQ ID NO: 8 Foldikine_cut237

DIKAHVNSLGENLKTLRLRLRRNHRFLPCENKSKAVEQVKNAFNKLQEKGIYKAMSEFDI
FINYIEAYMTM**N**NGGLDYLPNMLRDLRDAFSRVKTFFQMKDQLDNLLLKESLLEDFKGYL
GCQALSEMIQFYLEEVMPQAENQDPDIKAHVNSLGENLKTLRLRLRRNHRFLPCENKSKA
VEQVKNAFNKLQEKGIYKAMSEFDIFINYIEAYMTM**N**NGGLDYLPNMLRDLRDAFSRVKT
FFQMKDQLDNLLLKESLLEDFKGYLGCQALSEMIQFYLEEVMPQAENQDP

>SEQ ID NO: 9 ORKMCSF_013

KACVRTFYETPLQLLEKVKNVFNETKNLLDKDWNIFSKNCNNSFNECEDQDRRQPGTKTT
GKVSEYCSHMIGSGHLQSLQRLIDSQMETSCQITFEFVDQEQLKDPVCYLKKAFLLVQDI
MEDTMRFRDNTPNAIAIVQLQELSLRLKSCFTKDY

>SEQ ID NO: 10 ORKMCSF_014

KACVRTFYETPLQLLEKVKNVFNETKNLLDKDWNIFSKNCNNSF**A**ECGKPPQYIDRHTQPGQPNA**T**
SEYCSHMIGSGHLQSLQRLIDSQMETSCQITFEFVDQEQLKDPVCYLKKAFLLVQDIMEDTMRFRD
NTPNAIAIVQLQELSLRLKSCFTKDY

>SEQ ID NO: 11 IL10:

SPGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENKSKA
VEQVKNAFNKLQEKGIYKAMSEFDIFINYIEAYMTMKIRN

>SEQ ID NO: 50 IL10-HiBit:

SPGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENKSKA
VEQVKNAFNKLQEKGIYKAMSEFDIFINYIEAYMTMKIRN***GGVSGWRLFKKIS***

>SEQ ID NO: 12 CSF1

EEVSEYCSHMIGSGHLQSLQRLIDSQMETSCQITFEFVDQEQLKDPVCYLKKAFLLVQDI
MEDTMRFRDNTPNAIAIVQLQELSLRLKSCFTKDYEEHDKACVRTFYETPLQLLEKVKNV
FNETKNLLDKDWNIFSKNCNNSFAECSSQD

>SEQ ID NO: 51 CSF1-HiBit

EEVSEYCSHMIGSGHLQSLQRLIDSQMETSCQITFEFVDQEQLKDPVCYLKKAFLLVQDI
MEDTMRFRDNTPNAIAIVQLQELSLRLKSCFTKDYEEHDKACVRTFYETPLQLLEKVKNV
FNETKNLLDKDWNIFSKNCNNSFAECSSQD***GGVSGWRLFKKIS***

>SEQ ID NO: 13 IL-10-CSF1

```
SPGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENKSKA
VEQVKNAFNKLQEKGIYKAMSEFDIFINYIEAYMTMKIRNGGGGGSGGSGGSGGSGGSGG
SGGSGGSGGGEEVSEYCSHMIGSGHLQSLQRLIDSQMETSCQITFEFVDQEQLKDPVCYL
KKAFLLVQDIMEDTMRFRDNTPNAIAIVQLQELSLRLKSCFTKDYEEHDKACVRTFYETP
LQLLEKVKNVFNETKNLLDKDWNIFSKNCNNSFAECSSQD
```

>SEQ ID NO: 52 IL-10-CSF1-HiBit

```
SPGQGTQSENSCTHFPGNLPNMLRDLRDAFSRVKTFFQMKDQLDNLLLKESLLEDFKGYL
GCQALSEMIQFYLEEVMPQAENQDPDIKAHVNSLGENLKTLRLRLRRCHRFLPCENKSKA
VEQVKNAFNKLQEKGIYKAMSEFDIFINYIEAYMTMKIRNGGGGGSGGSGGSGGSGGSGG
SGGSGGSGGGEEVSEYCSHMIGSGHLQSLQRLIDSQMETSCQITFEFVDQEQLKDPVCYL
KKAFLLVQDIMEDTMRFRDNTPNAIAIVQLQELSLRLKSCFTKDYEEHDKACVRTFYETP
LQLLEKVKNVFNETKNLLDKDWNIFSKNCNNSFAECSSQDGGVSGWRLFKKIS
```

>SEQ ID NO: 14 Foldikine10-CSF1

```
MTQSENSCTHFPGNLPNMLRDLRDAFSRVKTFFQMKDQLDNLLLKESLLEDFKGYLGCQALSEMIQ
FYLEEVMPQAENQEPDIKAHVNSLGENLKTLRLRLRRCHRFLPCENKSKAVEQVKNAFNKLQEKGI
YKAMSEFDIFINYIEAYMTMNNGGLDYLPNMLRDLRDAFSRVKTFFQMKDQLDNLLLKESLLEDFK
GYLGCQALSEMIQFYLEEVMPQAENQDPDIKAHVNSLGENLKTLRLRLRRNHRFLPCENKSKAVEQ
VKNAFNKLQEKGIYKAMSEFDIFINYIEAYMTMKIRNGGGGGSGGSGGSGGSGGSGGSGGSGGSGG
GEEVSEYCSHMIGSGHLQSLQRLIDSQMETSCQITFEFVDQEQLKDPVCYLKKAFLLVQDIMEDTM
RFRDNTPNAIAIVQLQELSLRLKSCFTKDYEEHDKACVRTFYETPLQLLEKVKNVFNETKNLLDKD
WNIFSKNCNNSFAECSSQD
```

>SEQ ID NO: 53 Foldikine10-CSF1-HiBit

```
MTQSENSCTHFPGNLPNMLRDLRDAFSRVKTFFQMKDQLDNLLLKESLLEDFKGYLGCQALSEMIQ
FYLEEVMPQAENQEPDIKAHVNSLGENLKTLRLRLRRCHRFLPCENKSKAVEQVKNAFNKLQEKGI
YKAMSEFDIFINYIEAYMTMNNGGLDYLPNMLRDLRDAFSRVKTFFQMKDQLDNLLLKESLLEDFK
GYLGCQALSEMIQFYLEEVMPQAENQDPDIKAHVNSLGENLKTLRLRLRRNHRFLPCENKSKAVEQ
VKNAFNKLQEKGIYKAMSEFDIFINYIEAYMTMKIRNGGGGGSGGSGGSGGSGGSGGSGGSGGSGG
GEEVSEYCSHMIGSGHLQSLQRLIDSQMETSCQITFEFVDQEQLKDPVCYLKKAFLLVQDIMEDTM
RFRDNTPNAIAIVQLQELSLRLKSCFTKDYEEHDKACVRTFYETPLQLLEKVKNVFNETKNLLDKD
WNIFSKNCNNSFAECSSQDGGVSGWRLFKKIS
```

>SEQ ID NO: 15 CSF1-IL10

```
EEVSEYCSHMIGSGHLQSLQRLIDSQMETSCQITFEFVDQEQLKDPVCYLKKAFLLVQDI
MEDTMRFRDNTPNAIAIVQLQELSLRLKSCFTKDYEEHDKACVRTFYETPLQLLEKVKNV
FNETKNLLDKDWNIFSKNCNNSFAECSSQDGGGGGSGGSGGSGGSGGSGGSGGSGGSGGGGG
RASPGQGTQSENSCTHFPGNLPNMLRDLRDAFSRVKTFFQMKDQLDNLLLKESLLEDFKG
YLGCQALSEMIQFYLEEVMPQAENQDPDIKAHVNSLGENLKTLRLRLRRCHRFLPCENKS
KAVEQVKNAFNKLQEKGIYKAMSEFDIFINYIEAYMTMKIRN
```

>SEQ ID NO: 54 CSF1-IL10-HiBit

EEVSEYCSHMIGSGHLQSLQRLIDSQMETSCQITFEFVDQEQLKDPVCYLKKAFLLVQDI
MEDTMRFRDNTPNAIAIVQLQELSLRLKSCFTKDYEEHDKACVRTFYETPLQLLEKVKNV
FNETKNLLDKDWNIFSKNCNNSFAECSSQD*GGGGGSGGSGGSGGSGGSGGSGGSGGSGGG*
RASPGQGTQSENSCTHFPGNLPNMLRDLRDAFSRVKTFFQMKDQLDNLLLKESLLEDFKG
YLGCQALSEMIQFYLEEVMPQAENQDPDIKAHVNSLGENLKTLRLRLRRCHRFLPCENKS
KAVEQVKNAFNKLQEKGIYKAMSEFDIFINYIEAYMTMKIRN***GGVSGWRLFKKIS***

>SEQ ID NO: 16 CSF1-Foldikine10 (=CSF1-30aa-foldikine10)

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQD*GGGGGSGGSGGSGGSGGSGGSGGSGGSGGG*MTQSENSCTHFPGNLPNM
LRDLRDAFSRVKTFFQMKDQLDNLLLKESLLEDFKGYLGCQALSEMIQFYLEEVMPQAENQEPDIK
AHVNSLGENLKTLRLRLRRCHRFLPCENKSKAVEQVKNAFNKLQEKGIYKAMSEFDIFINYIEAYM
TMNNGGLDYLPNMLRDLRDAFSRVKTFFQMKDQLDNLLLKESLLEDFKGYLGCQALSEMIQFYLEE
VMPQAENQDPDIKAHVNSLGENLKTLRLRLRRNHRFLPCENKSKAVEQVKNAFNKLQEKGIYKAMS
EFDIFINYIEAYMTMKIRN

>SEQ ID NO: 55 CSF1-Foldikine10-HiBit (=CSF1-30aa-foldikine10 -HiBit)

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQD*GGGGGSGGSGGSGGSGGSGGSGGSGGSGGG*MTQSENSCTHFPGNLPNM
LRDLRDAFSRVKTFFQMKDQLDNLLLKESLLEDFKGYLGCQALSEMIQFYLEEVMPQAENQEPDIK
AHVNSLGENLKTLRLRLRRCHRFLPCENKSKAVEQVKNAFNKLQEKGIYKAMSEFDIFINYIEAYM
TMNNGGLDYLPNMLRDLRDAFSRVKTFFQMKDQLDNLLLKESLLEDFKGYLGCQALSEMIQFYLEE
VMPQAENQDPDIKAHVNSLGENLKTLRLRLRRNHRFLPCENKSKAVEQVKNAFNKLQEKGIYKAMS
EFDIFINYIEAYMTMKIRN***GGVSGWRLFKKIS***

>SEQ ID NO: 17 foldikine10_cut116-CSF1

NKSKAVEQVKNAFNKLQEKGIYKAMSEFDIFINYIEAYMTMNNGGLDYLPNMLRDLRDAFSRVKTF
FQMKDQLDNLLLKESLLEDFKGYLGCQALSEMIQFYLEEVMPQAENQDPDIKAHVNSLGENLKTLR
LRLRRNHRFLPCENKSKAVEQVKNAFNKLQEKGIYKAMSEFDIFINYIEAYMTMNNGGLDYLPNML
RDLRDAFSRVKTFFQMKDQLDNLLLKESLLEDFKGYLGCQALSEMIQFYLEEVMPQAENQDPDIKA
HVNSLGENLKTLRLRLRRNHRFLPCE*GGGGGSGGSGGSGGSGGSGGSGGSGGSGGG*EEVSEYCSHM
IGSGHLQSLQRLIDSQMETSCQITFEFVDQEQLKDPVCYLKKAFLLVQDIMEDTMRFRDNTPNAIA
IVQLQELSLRLKSCFTKDYEEHDKACVRTFYETPLQLLEKVKNVFNETKNLLDKDWNIFSKNCNNS
FAECSSQD

>SEQ ID NO: 56 foldikine10_cut116-CSF1-HiBit

NKSKAVEQVKNAFNKLQEKGIYKAMSEFDIFINYIEAYMTMNNGGLDYLPNMLRDLRDAFSRVKTF
FQMKDQLDNLLLKESLLEDFKGYLGCQALSEMIQFYLEEVMPQAENQDPDIKAHVNSLGENLKTLR
LRLRRNHRFLPCENKSKAVEQVKNAFNKLQEKGIYKAMSEFDIFINYIEAYMTMNNGGLDYLPNML
RDLRDAFSRVKTFFQMKDQLDNLLLKESLLEDFKGYLGCQALSEMIQFYLEEVMPQAENQDPDIKA
HVNSLGENLKTLRLRLRRNHRFLPCE*GGGGGSGGSGGSGGSGGSGGSGGSGGSGGG*EEVSEYCSHM

IGSGHLQSLQRLIDSQMETSCQITFEFVDQEQLKDPVCYLKKAFLLVQDIMEDTMRFRDNTPNAIA
IVQLQELSLRLKSCFTKDYEEHDKACVRTFYETPLQLLEKVKNVFNETKNLLDKDWNIFSKNCNNS
FAECSSQD***GGGVSGWRLFKKIS***

>SEQ ID NO: 18 CSF1-foldikine10_cut116

MTAPGAAGRCPPTTWLGSLLLLVCLLASRSITEEVSEYCSHMIGSGHLQSLQRLIDSQME
TSCQITFEFVDQEQLKDPVCYLKKAFLLVQDIMEDTMRFRDNTPNAIAIVQLQELSLRLK
SCFTKDYEEHDKACVRTFYETPLQLLEKVKNVFNETKNLLDKDWNIFSKNCNNSFAECSS
QDGGGGGSGGSGGSGGSGGSGGSGGSGGSGGSGGGMTQSENSCTHFPGNLPNMLRDLRDAFSR
VKTFFQMKDQLDNLLLKESLLEDFKGYLGCQALSEMIQFYLEEVMPQAENQEPDIKAHVN
SLGENLKTLRLRLRRCHRFLPCENKSKAVEQVKNAFNKLQEKGIYKAMSEFDIFINYIEA
YMTMNNGGLDYLPNMLRDLRDAFSRVKTFFQMKDQLDNLLLKESLLEDFKGYLGCQALSE
MIQFYLEEVMPQAENQDPDIKAHVNSLGENLKTLRLRLRRNHRFLPCENKSKAVEQVKNA
FNKLQEKGIYKAMSEFDIFINYIEAYMTMKIRN

>SEQ ID NO: 57 CSF1-foldikine10_cut116-HiBit

MTAPGAAGRCPPTTWLGSLLLLVCLLASRSITEEVSEYCSHMIGSGHLQSLQRLIDSQME
TSCQITFEFVDQEQLKDPVCYLKKAFLLVQDIMEDTMRFRDNTPNAIAIVQLQELSLRLK
SCFTKDYEEHDKACVRTFYETPLQLLEKVKNVFNETKNLLDKDWNIFSKNCNNSFAECSS
QDGGGGGSGGSGGSGGSGGSGGSGGSGGSGGSGGGMTQSENSCTHFPGNLPNMLRDLRDAFSR
VKTFFQMKDQLDNLLLKESLLEDFKGYLGCQALSEMIQFYLEEVMPQAENQEPDIKAHVN
SLGENLKTLRLRLRRCHRFLPCENKSKAVEQVKNAFNKLQEKGIYKAMSEFDIFINYIEA
YMTMNNGGLDYLPNMLRDLRDAFSRVKTFFQMKDQLDNLLLKESLLEDFKGYLGCQALSE
MIQFYLEEVMPQAENQDPDIKAHVNSLGENLKTLRLRLRRNHRFLPCENKSKAVEQVKNA
FNKLQEKGIYKAMSEFDIFINYIEAYMTMKIRN**GGVSGWRLFKKIS**

>SEQ ID NO: 19 CSF1-25aa-foldikine10

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGSGGSGGSGGSGGSGGSGGSGGGMTQSENSCTHFPGNLPNM
LRDLRDAFSRVKTFFQMKDQLDNLLLKESLLEDFKGYLGCQALSEMIQFYLEEVMPQAENQEPDIK
AHVNSLGENLKTLRLRLRRCHRFLPCENKSKAVEQVKNAFNKLQEKGIYKAMSEFDIFINYIEAYM
TMNNGGLDYLPNMLRDLRDAFSRVKTFFQMKDQLDNLLLKESLLEDFKGYLGCQALSEMIQFYLEE
VMPQAENQDPDIKAHVNSLGENLKTLRLRLRRNHRFLPCENKSKAVEQVKNAFNKLQEKGIYKAMS
EFDIFINYIEAYMTMKIRN

>SEQ ID NO: 58 CSF1-25aa-foldikine10-HiBit

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGSGGSGGSGGSGGSGGSGGSGGGMTQSENSCTHFPGNLPNM
LRDLRDAFSRVKTFFQMKDQLDNLLLKESLLEDFKGYLGCQALSEMIQFYLEEVMPQAENQEPDIK
AHVNSLGENLKTLRLRLRRCHRFLPCENKSKAVEQVKNAFNKLQEKGIYKAMSEFDIFINYIEAYM
TMNNGGLDYLPNMLRDLRDAFSRVKTFFQMKDQLDNLLLKESLLEDFKGYLGCQALSEMIQFYLEE
VMPQAENQDPDIKAHVNSLGENLKTLRLRLRRNHRFLPCENKSKAVEQVKNAFNKLQEKGIYKAMS
EFDIFINYIEAYMTMKIRN**GGVSGWRLFKKIS**

>SEQ ID NO: 21 CSF1-35aa-foldikine10

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGGGSGGSGGSGGSGGSGGSGGSGGGMTQSENSCTHFPG
NLPNMLRDLRDAFSRVKTFFQMKDQLDNLLLKESLLEDFKGYLGCQALSEMIQFYLEEVMPQAENQ
EPDIKAHVNSLGENLKTLRLRLRRCHRFLPCENKSKAVEQVKNAFNKLQEKGIYKAMSEFDIFINY

IEAYMTMNNGGLDYLPNMLRDLRDAFSRVKTFFQMKDQLDNLLLKESLLEDFKGYLGCQALSEMIQ
FYLEEVMPQAENQDPDIKAHVNSLGENLKTLRLRLRRNHRFLPCENKSKAVEQVKNAFNKLQEKGI
YKAMSEFDIFINYIEAYMTMKIRN

>SEQ ID NO: 59 CSF1-35aa-foldikine10-HiBit

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQD*GGGGGSGGGGSGGSGGSGGSGGSGGSGGSGGSGGG*MTQSENSCTHFPG
NLPNMLRDLRDAFSRVKTFFQMKDQLDNLLLKESLLEDFKGYLGCQALSEMIQFYLEEVMPQAENQ
EPDIKAHVNSLGENLKTLRLRLRRCHRFLPCENKSKAVEQVKNAFNKLQEKGIYKAMSEFDIFINY
IEAYMTMNNGGLDYLPNMLRDLRDAFSRVKTFFQMKDQLDNLLLKESLLEDFKGYLGCQALSEMIQ
FYLEEVMPQAENQDPDIKAHVNSLGENLKTLRLRLRRNHRFLPCENKSKAVEQVKNAFNKLQEKGI
YKAMSEFDIFINYIEAYMTMKIRN***GGVSGWRLFKKIS***

>SEQ ID NO: 22 CSF1-15aa-foldikine10

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQD*GGSGGSGGSGGSGGG*MTQSENSCTHFPGNLPNMLRDLRDAFSRVKTFF
QMKDQLDNLLLKESLLEDFKGYLGCQALSEMIQFYLEEVMPQAENQEPDIKAHVNSLGENLKTLRL
RLRRCHRFLPCENKSKAVEQVKNAFNKLQEKGIYKAMSEFDIFINYIEAYMTMNNGGLDYLPNMLR
DLRDAFSRVKTFFQMKDQLDNLLLKESLLEDFKGYLGCQALSEMIQFYLEEVMPQAENQDPDIKAH
VNSLGENLKTLRLRLRRNHRFLPCENKSKAVEQVKNAFNKLQEKGIYKAMSEFDIFINYIEAYMTM
KIRN

>SEQ ID NO: 60 CSF1-15aa-foldikine10-HiBit

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQD*GGSGGSGGSGGSGGG*MTQSENSCTHFPGNLPNMLRDLRDAFSRVKTFF
QMKDQLDNLLLKESLLEDFKGYLGCQALSEMIQFYLEEVMPQAENQEPDIKAHVNSLGENLKTLRL
RLRRCHRFLPCENKSKAVEQVKNAFNKLQEKGIYKAMSEFDIFINYIEAYMTMNNGGLDYLPNMLR
DLRDAFSRVKTFFQMKDQLDNLLLKESLLEDFKGYLGCQALSEMIQFYLEEVMPQAENQDPDIKAH
VNSLGENLKTLRLRLRRNHRFLPCENKSKAVEQVKNAFNKLQEKGIYKAMSEFDIFINYIEAYMTM
KIRN***GGVSGWRLFKKIS***

>SEQ ID NO: 23 CSF1-15aa-IL10

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQD*GGSGGSGGSGGSGGG*RASPGQGTQSENSCTHFPGNLPNMLRDLRDAFS
RVKTFFQMKDQLDNLLLKESLLEDFKGYLGCQALSEMIQFYLEEVMPQAENQDPDIKAHVNSLGEN
LKTLRLRLRRCHRFLPCENKSKAVEQVKNAFNKLQEKGIYKAMSEFDIFINYIEAYMTMKIRN

>SEQ ID NO: 61 CSF1-15aa-IL10-HiBit

EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQD*GGSGGSGGSGGSGGG*RASPGQGTQSENSCTHFPGNLPNMLRDLRDAFS
RVKTFFQMKDQLDNLLLKESLLEDFKGYLGCQALSEMIQFYLEEVMPQAENQDPDIKAHVNSLGEN
LKTLRLRLRRCHRFLPCENKSKAVEQVKNAFNKLQEKGIYKAMSEFDIFINYIEAYMTMKIRN***GGV***
***SGWRLFKKIS***

>SEQ ID NO: 24 CSF1-25aa-IL10

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGSGGSGGSGGSGGSGGSGGSGGGRASPGQGTQSENSCTHFPGNLPN
MLRDLRDAFSRVKTFFQMKDQLDNLLLKESLLEDFKGYLGCQALSEMIQFYLEEVMPQAENQDPDI

KAHVNSLGENLKTLRLRLRRCHRFLPCENKSKAVEQVKNAFNKLQEKGIYKAMSEFDIFINYIEAY
MTMKIRN
```

>SEQ ID NO: 62 CSF1-25aa-IL10-HiBit

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGSGGSGGSGGSGGSGGSGGSGGGRASPGQGTQSENSCTHFPGNLPN
MLRDLRDAFSRVKTFFQMKDQLDNLLLKESLLEDFKGYLGCQALSEMIQFYLEEVMPQAENQDPDI
KAHVNSLGENLKTLRLRLRRCHRFLPCENKSKAVEQVKNAFNKLQEKGIYKAMSEFDIFINYIEAY
MTMKIRNGGVSGWRLFKKIS
```

>SEQ ID NO: 25 CSF1-30aa-IL10

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGSGGSGGSGGSGGSGGSGGGGRASPGQGTQSENSCTHFP
GNLPNMLRDLRDAFSRVKTFFQMKDQLDNLLLKESLLEDFKGYLGCQALSEMIQFYLEEVMPQAEN
QDPDIKAHVNSLGENLKTLRLRLRRCHRFLPCENKSKAVEQVKNAFNKLQEKGIYKAMSEFDIFIN
YIEAYMTMKIRN
```

>SEQ ID NO: 63 CSF1-30aa-IL10-HiBit

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGSGGSGGSGGSGGSGGSGGGGRASPGQGTQSENSCTHFP
GNLPNMLRDLRDAFSRVKTFFQMKDQLDNLLLKESLLEDFKGYLGCQALSEMIQFYLEEVMPQAEN
QDPDIKAHVNSLGENLKTLRLRLRRCHRFLPCENKSKAVEQVKNAFNKLQEKGIYKAMSEFDIFIN
YIEAYMTMKIRNGGVSGWRLFKKIS
```

>SEQ ID NO: 26 CSF1-35aa-IL10

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGGGSGGSGGSGGSGGSGGSGGSGGGRASPGQGTQSENS
CTHFPGNLPNMLRDLRDAFSRVKTFFQMKDQLDNLLLKESLLEDFKGYLGCQALSEMIQFYLEEVM
PQAENQDPDIKAHVNSLGENLKTLRLRLRRCHRFLPCENKSKAVEQVKNAFNKLQEKGIYKAMSEF
DIFINYIEAYMTMKIRN
```

>SEQ ID NO: 64 CSF1-35aa-IL10-HiBit

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGGGSGGSGGSGGSGGSGGSGGSGGGRASPGQGTQSENS
CTHFPGNLPNMLRDLRDAFSRVKTFFQMKDQLDNLLLKESLLEDFKGYLGCQALSEMIQFYLEEVM
PQAENQDPDIKAHVNSLGENLKTLRLRLRRCHRFLPCENKSKAVEQVKNAFNKLQEKGIYKAMSEF
DIFINYIEAYMTMKIRNGGVSGWRLFKKIS
```

>SEQ ID NO: 27 repeated unit
GGSGG

>SEQ ID NO: 28

**M**TQSENSCTHFPGNLPNMLRDLRDAFSRVKTFFQMKDQLDNLLLKESLLEDFKGYLGCQALSEMIQ
FYLEEVMPQAENQ**E**PDIKAHVNSLGENLKTLRLRLRRCHRFLPCENKSKAVEQVKNAFNKLQEKGI
YKAMSEFDIFINYIEAYMTM**N**

>SEQ ID NO: 29

LPNMLRDLRDAFSRVKTFFQMKDQLDNLLLKESLLEDFKGYLGCQALSEMIQFYLEEVMPQAENQD
PDIKAHVNSLGENLKTLRLRLRRCHRFLPCENKSKAVEQVKNAFNKLQEKGIYKAMSEFDIFINYI
EAYMTMKIRN

>SEQ ID NO: 30 Nt Ct linker
NGGLDY

>SEQ ID NO: 31 Nt Ct linker
YKTIT

>SEQ ID NO: 32 Nt Ct linker
DKDIRDGD

>SEQ ID NO: 33 intermolecular linker 1 15
GGSGGSGGS GSGGG

>SEQ ID NO: 34 intermolecular linker 1 25
GGSGGSGGS GGSGGSGGSG GSGGG

>SEQ ID NO: 35 intermolecular linker 1 30
GGGGSGGSG GSGGSGGSGG SGGSGGSGGG

>SEQ ID NO: 36 intermolecular linker 1 35
GGGGGSGGGG SGGSGGSGGS GGSGGSGGSG GSGGG

>SEQ ID NO:37 1 136

CTHFPGNLPNMLRDLRDAFSRVKTFFQMKDQLDNLLLKESLLEDFKGYLGCQALSEMIQFYLEEVM
PQAENQ_D_PDIKAHVNSLGENLKTLRLRLRRCHRFLPCENKSKAVEQVKNAFNKLQEKGIYKAMSEF
DIFINY_I_EA

>SEQ ID NO: 38

NMLRDLRDAFSRVKTFFQMKDQLDNLLLKESLLEDFKGYLGCQALSEMIQFYLEEVMPQAENQDPD
IKAHVNSLGENLKTLRLRLRRCHRFLPCENKSKAVEQVKNAFNKLQEKGIYKAMSEFDIFINYIEA
YMTM

>SEQ ID NO: 39

**M**TQSENSCTHFOGNLPNMLRDLRDAFSRVKTFFQMKDQLDNLLLKESLLEDFKGYLGCQALSEMIQ
FYLEEVMPQAENQDPDIKAHVNSLGENLKTLRLRLRRCHRFLPCENKSKAVEQVKNAFNKLQEKGI
YKAMSEFDIFINYIEAMTM**N**

>SEQ ID NO: 40

LPNMLRDLRDAFSRVKTFFQMKDQLDNLLLKESLLEDFKGYLGCQALSEMIQFYLEEVMPQAENQD
PDIKAHVNSLGENLKTLRLRLRRCHRFLPCENKSKAVEQVKNAFNKLQEKGIYKAMSEFDIFINYI
EAYMTM

>SEQ ID NO: 41

**M**TQSENSCTHFOGNLPNMLRDLRDAFSRVKTFFQMKDQLDNLLLKESLLEDFKGYLGCQALSEMIQ
FYLEEVMPQAENQDPDIKAHVNSLGENLKTLRLRLRRCHRFLPCENKSKAVEQVKNAFNKLQEKGI
YKAMSEFDIFINYIEAY**L**

>SEQ ID NO: 42

**PEFA**NMLRDLRDAFSRVKTFFQMKDQLDNLLLKESLLEDFKGYLGCQALSEMIQFYLEEVMPQAEN
QDPDIKAHVNSLGENLKTLRLRLRRCHRFLPCENKSKAVEQVKNAFNKLQEKGIYKAMSEFDIFIN
YIEAYMTM

>SEQ ID NO: 43

**M**TQSENSCTHFPGNLPNMLRDLRDAFSRVKTFFQMKDQLDNLLLKESLLEDFKGYLGCQALSEMIQ
FYLEEVMPQAENQDPDIKAHVNSLGENLKTLRLRLRRCHRFLPCENKSKAVEQVKNAFNKLQEKGI
YKAMSEFDIFINYIEA**K**

>SEQ ID NO: 44

**ELA**NMLRDLRDAFSRVKTFFQMKDQLDNLLLKESLLEDFKGYLGCQALSEMIQFYLEEVMPQAENQ
DPDIKAHVNSLGENLKTLRLRLRRCHRFLPCENKSKAVEQVKNAFNKLQEKGIYKAMSEFDIFINY
IEAYMTM

>SEQ ID NO: 71
NNGGLDYL

>SEQ ID NO: 45
NFGGLDYL

>SEQ ID NO: 46
LYKTITPEFA

>SEQ ID NO: 47
KDKDIRDGDELA

>SEQ ID NO: 48
FGGLDY

>SEQ ID NO: 65 N-terminal part of SC dimeric IL-10

MTQSENSCTHFPGNLPNMLRDLRDAFSRVKTFFQMKDQLDNLLLKESLLEDFKGYLGCQALSEMIQ
FYLEEVMPQAENQDPDIKAHVNSLGENLKTLRLRLRRCHRFLPCENKSKAVEQVKNAFNKLQEKGI
YKAMSEFDIFINYIEAY

>SEQ ID NO: 66 C-terminal part of SC dimeric IL-10

NMLRDLRDAFSRVKTFFQMKDQLDNLLLKESLLEDFKGYLGCQALSEMIQFYLEEVMPQAENQDPD
IKAHVNSLGENLKTLRLRLRRCHRFLPCENKSKAVEQVKNAFNKLQEKGIYKAMSEFDIFINYIEA
YMTMKIRN

>SEQ ID NO: 67
MTMN

>SEQ ID NO: 68
PEFA

>SEQ ID NO: 72 CSF1-Foldikine10-TEV-his-HiBit

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGSGGSGGSGGSGGSGGSGGSGGGMTQSENSCTHFPGNLPNM
LRDLRDAFSRVKTFFQMKDQLDNLLLKESLLEDFKGYLGCQALSEMIQFYLEEVMPQAENQEPDIK
AHVNSLGENLKTLRLRLRRCHRFLPCENKSKAVEQVKNAFNKLQEKGIYKAMSEFDIFINYIEAYM
TMNNGGLDYLPNMLRDLRDAFSRVKTFFQMKDQLDNLLLKESLLEDFKGYLGCQALSEMIQFYLEE

VMPQAENQDPDIKAHVNSLGENLKTLRLRLRRNHRFLPCENKSKAVEQVKNAFNKLQEKGIYKAMS
EFDIFINYIEAYMTMKIRN**GENLYFQSGGHHHHHHGGGVSGWRLFKKIS***
```

>SEQ ID NO: 73 CSF1(E82A)-Foldikine10-TEV-his-HiBit

```
EEVSEYCSHMIGSGHLQSLQRLIDSQMETSCQITFEFVDQEQLKDPVCYLKKAFLLVQDIMEDTMR
FRDNTPNAIAIVQLQ**A**LSLRLKSCFTKDYEEHDKACVRTFYETPLQLLEKVKNVFNETKNLLDKDW
NIFSKNCNNSFAECSSQDGGGGGSGGSGGSGGSGGSGGSGGSGGSGGGMTQSENSCTHFPGNLPNM
LRDLRDAFSRVKTFFQMKDQLDNLLLKESLLEDFKGYLGCQALSEMIQFYLEEVMPQAENQEPDIK
AHVNSLGENLKTLRLRLRRCHRFLPCENKSKAVEQVKNAFNKLQEKGIYKAMSEFDIFINYIEAYM
TMNNGGLDYLPNMLRDLRDAFSRVKTFFQMKDQLDNLLLKESLLEDFKGYLGCQALSEMIQFYLEE
VMPQAENQDPDIKAHVNSLGENLKTLRLRLRRNHRFLPCENKSKAVEQVKNAFNKLQEKGIYKAMS
EFDIFINYIEAYMTMKIRN**GENLYFQSGGHHHHHHGGGVSGWRLFKKIS***
```

>SEQ ID NO: 74 CSF1-IL10-TEV-his-HiBit

```
EEVSEYCSHMIGSGHLQSLQRLIDSQMETSCQITFEFVDQEQLKDPVCYLKKAFLLVQDIMEDTMR
FRDNTPNAIAIVQLQELSLRLKSCFTKDYEEHDKACVRTFYETPLQLLEKVKNVFNETKNLLDKDW
NIFSKNCNNSFAECSSQDGGGGGSGGSGGSGGSGGSGGSGGSGGSGGGSPGQGTQSENSCTHFPGN
LPNMLRDLRDAFSRVKTFFQMKDQLDNLLLKESLLEDFKGYLGCQALSEMIQFYLEEVMPQAENQD
PDIKAHVNSLGENLKTLRLRLRRCHRFLPCENKSKAVEQVKNAFNKLQEKGIYKAMSEFDIFINYI
EAYMTMKIRN**GENLYFQSGGHHHHHHGGGVSGWRLFKKIS***
```

**Claims**

1. A single chain polypeptide having IL-10 and CSF1 activities.

2. The single chain polypeptide according to claim 1, which comprises in the N-terminus to C-terminus direction, a CSF1 monomer, a peptide linker, and an IL-10 monomer.

3. The single chain polypeptide according to claim 1 or 2, which comprises sequence SEQ ID NO: 24, SEQ ID NO: 25, SEQ ID NO: 26, or a sequence at least 80% identical thereto and that retains IL-10 and CSF1 activities.

4. The single chain polypeptide according to claim 1, which comprises a single chain dimeric IL-10 fused to a CSF1 monomer, in particular single chain dimeric IL-10-CSF1 monomer fusion protein, or a CSF1 monomer-single chain dimeric IL-10 fusion protein.

5. The single chain polypeptide according to any one of claims 1 to 4, wherein the CSF1 monomer is a wild-type CSF1 monomer, or a mutant thereof, or a circular permutant CSF1 monomer.

6. The single chain polypeptide according to any one of claims 1 to 5, which has similar or higher affinity to CSF1R compared to wild-type CSF1.

7. The single chain polypeptide according to any one of claims 1 to 6, wherein the CSF1 monomer is a CSF1 monomer mutant that comprises sequence SEQ ID NO:12 modified by at least the substitutions:

   a) Q17R;
   b) V78W;
   c) T124I;
   d) V120I;
   e) Q17R, T124I and V120I;
   f) V78W, T124I and V120I; or
   g) Q17R, V78W, T124I and V120I.

8. The single chain polypeptide according to any one of claims 1 to 6, wherein the CSF1 monomer is a CSF1 circular permutant:

   a) generated by linking an amino acid in each of the N- and C-terminal regions of CSF-1 and by suppressing the peptide bond consisting of residues at positions 95 to 99 or SEQ ID NO: 12; or
   b) that comprises sequence SEQ ID NO: 9, or SEQ ID NO: 10, or a sequence at least 80% identical thereto and that retains CSF1 activity.

9. The single chain polypeptide according to any one of claims 1 and 4 to 8, wherein the single chain dimeric IL-10 is a fusion protein comprising a first IL-10 monomer fragment comprising at least $\alpha$-helices A to F of IL-10, a peptide linker, and a second IL-10 monomer fragment comprising at least $\alpha$-helices A to F of IL-10, or a circular permutant thereof.

10. The single chain polypeptide according to any one of claims 1 and 4 to 9, wherein the single chain dimeric IL-10 sequence SEQ ID NO: 65-X-(NtCt)-Z-SEQ ID NO: 66,

    wherein (NtCt) is a peptide linker,
    wherein X is absent or present, and where present it consists in one or more amino acids of the original IL-10 sequence in continuity with the preceding amino acids on its N-terminal side, optionally with a mutation to accommodate the NtCt linker, wherein Z is absent or present, and where present it consists one or more amino acids of the original IL-10 sequence in continuity with the preceding amino acids on its C-terminal side, optionally with a mutation to accommodate the NtCt linker, or a sequence at least 80%, at least 85%, at least 90%, at least 95%, at least 98%, at least 99% or 100% identical thereto and that retain at least the same stability, and/or at least the same level of interaction with IL-10 receptor.

11. The single chain polypeptide according to claim 9 or 10, wherein the peptide linker comprises from 3 to 20 amino acid residues and comprises no more than 2 adjacent Gly and/or Ser residues.

12. The single chain polypeptide according to any one of claims 1 and 4 to 11, wherein the single chain dimeric IL-10 comprises SEQ ID NO: 1, SEQ ID NO: 2, SEQ ID NO: 3, or SEQ ID NO: 4, or a sequence at least 80% identical thereto and that retains IL-10 activity.

13. The single chain polypeptide according to any one of claims 1 and 4 to 8, wherein the single chain dimeric IL-10 is a circular permutant:

    a) comprising a first IL-10 monomer fragment comprising $\alpha$-helices E to F of IL-10, a first peptide linker, and a second IL-10 monomer fragment comprising at least $\alpha$-helices A to F of IL-10, a second peptide linker, and a third IL-10 monomer fragment comprising at least $\alpha$-helices A to D of IL-10, and/or
    b) comprising sequence SEQ ID NO: 5, or a sequence at least 80% identical thereto and that retains IL-10 activity.

14. The single chain polypeptide according to any one of claims 1 and 4 to 13, wherein the single chain dimeric IL-10 and CSF1 monomer are linked through a linker that comprises from 12 to 40 amino acid residues.

15. The single chain polypeptide according to any one of claims 1 and 4 to 14, which comprises sequence SEQ ID NO: 14, SEQ ID NO: 16, SEQ ID NO: 17, SEQ ID NO: 18, SEQ ID NO: 19, SEQ ID NO: 1 , SEQ ID NO: 21, SEQ ID NO: 22, or a sequence at least 80% identical thereto and that retains IL-10 and CSF1 activities.

16. The single chain polypeptide according to any one of claims 1 to 15, which has reduced affinity to IL-10 receptor α (IL10RA) and IL-10 receptor β (IL10RB) compared to wild-type IL-10.

17. A pharmaceutical composition comprising the single chain polypeptide according to any one of claims 1 to 16 and a pharmaceutically acceptable carrier.

18. A single chain polypeptide according to any one of claims 1 to 16, for use as a medicament, in particular for use for treating an inflammatory disease.

# Decoupling pro- and anti-inflammatory functions of IL-10

Preferential delivery of IL-10 and CSF-1 to myeloid cells vs lymphoid cells

FIG.1

FIG.2

FIG.3

Foldikine 10

Foldikine_cut116

Foldikine_cut133

Foldikine_cut209

Foldikine_cut237

FIG.4

EP 4 755 906 A2

FIG.5

FIG.6

**Circular permutants CSF1**

FIG.7

FIG.8

HEK reporter IL-10R

FIG.9

FIG.10

**HEK reporter CSF1R**

FIG.11

Biological replica 1

Biological replica 2

A

CSF1     IL10        CSF1     IL10

B

CSF1     Foldikine10     CSF1     Foldikine10

FIG.12 (Beginning)

FIG.12 (End)

FIG.13

EP 4 755 906 A2

FIG.14

**HEK reporter IL-10R**

**HEK reporter CSF1R**

EP 4 755 906 A2

## HEK reporter IL-10R

FIG.15

## HEK reporter CSF1R

FIG.16

FIG.17

FIG.18

FIG.19

EP 4 755 906 A2

FIG.20 (Beginning)

C

TNF-α

FIG.20 (End)

FIG.21 (Beginning)

C

CD163

CD86

FIG.21 (End)

Untreated
rIL10
rCSF1
FDLK1

FIG.22

FIG.23

FIG.24 (Beginning)

C

FIG.24 (End)

FIG.25 (Beginning)

C

GranzymeB

FIG.25 (End)

FIG.26 (Beginning)

C

FIG.26 (End)

A

B

FIG.27 (Beginning)

C

FIG.27 (End)

A

B

FIG.28

A

B

## FIG.29

**EP 4 755 906 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2023052202 W

**Non-patent literature cited in the description**

- Remington's Pharmaceutical Sciences. Mack Publishing Co., 1995, 1447-1676
- **MONTERO-BLAY et al.** *Molecular Systems Biology*, 2023, vol. 19, e11037
- **SCHYMKOWITZ et al.** *PNAS*, 2005, vol. 102 (29), 10147-10152
- **GIBSON DG et al.** *Nat Methods.*, May 2009, vol. 6 (5), 343-5